(19) Europäisches Patentamt
European Patent Office
Office européen des brevets

(11) Numéro de publication: **0 063 525**
**A1**

# (12) DEMANDE DE BREVET EUROPEEN

(21) Numéro de dépôt: 82400680.3

(22) Date de dépôt: 15.04.82

(51) Int. Cl.³: **C 07 D 498/04**
C 07 D 487/04, C 07 D 491/14
C 07 D 491/22, A 61 K 31/55
//(C07D498/04, 261/00, 223/00),
(C07D487/04, 231/00, 223/00)

(30) Priorité: 16.04.81 FR 8107707

(43) Date de publication de la demande: 27.10.82 Bulletin 82/43

(84) Etats contractants désignés: BE CH DE FR GB IT LI NL SE

(71) Demandeur: CENTRE NATIONAL DE LA RECHERCHE SCIENTIFIQUE
15, Quai Anatole France
F-75007 Paris(FR)

(72) Inventeur: Viel, Claude
2 Rue des Bleuets
F-94140 Alfortville(FR)

(72) Inventeur: Marçot, Bernoud
61 Avenue d'Assas
F-78470 St. Rémy les Chevreuse(FR)

(72) Inventeur: Redeuilh, Gérard
21 Bld. Saint Martin
F-75003 Paris(FR)

(72) Inventeur: Djiane, Alain
105 Avenue du Roule
F-92200 Neuilly/Seine(FR)

(72) Inventeur: Cherqui, Jean
6 Bld. Suchet
F-75016 Paris(FR)

(74) Mandataire: Burtin, Jean-François
5 Bis, rue Parmentier
F-92200 Neuilly S/Seine(FR)

## (54) Nouveaux dérivés tétracycliques de la dibenzazépine, leur procédé de préparation et les compositions pharmaceutiques en renfermant.

(57) Dérivés tétracycliques de la dibenzazépine, applicables notamment en tant que principe actif de médicaments antidépresseurs, étant caractérisés par la formule

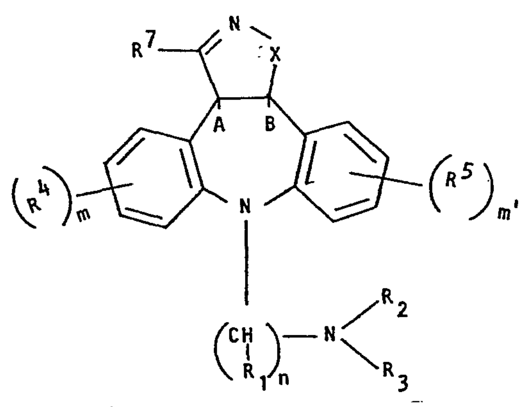

dans laquelle $R_1$ représente hydrogène ou alcoyle, n un nombre entier variant de 1 à 5,

$R_2$ représente hydrogène, alcoyle, aralcoyle ou alcényle,

$R_3$ représente hydrogène, alcoyle, aralcoyle ou alcényle ou bien

$R_2$ et $R_3$, ensemble, forment une chaîne alcoylène, éventuellement interrompue par 1 ou 2 hétéroatomes choisis dans le groupe constitué par un oxygène, un soufre et le radical N-$R_6$ ($R_6$ est de l'hydrogène, alcoyle, hydroxyalcoyle, alcoxyalcoyle ou acyloxyalcoyle),

$R_4$ et $R_5$, distinctement l'un de l'autre, sont hydrogène, alcoyle, halogène, trifluorométhyle, alcoxy acyloxylènedioxy, hydroxy, thio, alcoyl thio, trichlorométhoxy, trifluorométhoxy, trifluorométhyl thio, amino, alcoylamino, arylamino, alcoylamino sulfonyle, morpholino sulfonyle, aminosulfonyle, cyano, nitro, carboxy, alcoyloxycarbonyl, carbonamido, sulfinyle, sulfonyle, formyle ou radical acyle,

$R_7$ est alcoyle, phényle éventuellement substitué par $R_4$, m et m', distinctement l'un de l'autre, varient de 1 à 3, A et B symbolisent 2 atomes d'hydrogène ou une double liaison carbone-carbone,

X représente oxygène, ou groupe N-$R_8$ ($R_8$ étant un phényle éventuellement substitué par un, deux ou trois substituants, ou alcoyle).

EP 0 063 525 A1

NOUVEAUX DERIVES TETRACYCLIQUES DE LA DIBENZAZEPINE, LEUR PROCEDE DE PREPARATION ET LES COMPOSITIONS PHARMACEUTIQUES EN RENFERMANT.

La présente invention a pour objet de nouveaux dérivés de la dibenzazépine, leur procédé de préparation et les compositions pharmaceutiques en renfermant.

Elle a plus particulièrement pour objet des dibenzazépines dont l'atome d'azote est substitué par une chaîne amino-alcoylène.

Elle a spécifiquement pour objet des dérivés tétracycliques de dibenzazépines, répondant à la formule générale I

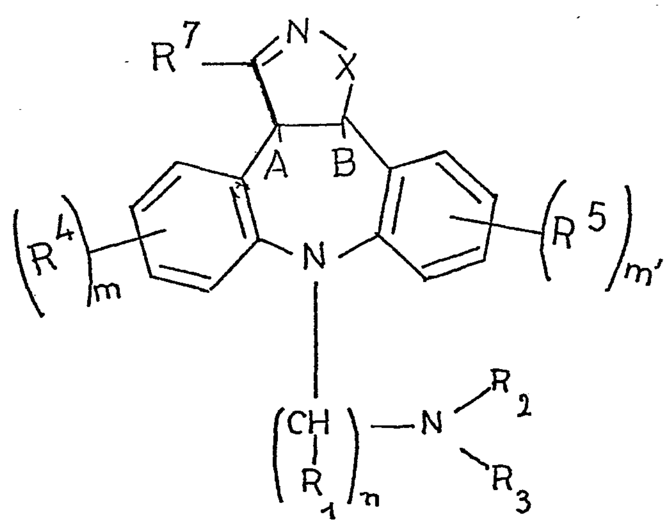

(I)

dans laquelle :

- A et B symbolisent 2 atomes d'hydrogène ou une double liaison carbone-carbone,
- X représente de l'oxygène, ou le groupe N-$R_8$ ($R_8$ étant un radical phényle ou phényle substitué par un, deux ou trois substituants, ou un radical alcoyle inférieur) ;

$R_1$ représente de l'hydrogène ou un radical alcoyle inférieur,

$R_2$ représente de l'hydrogène, un radical alcoyle inférieur, un radical aralcoyle inférieur ou alcényle inférieur,

$R_3$ représente de l'hydrogène, un radical alcoyle inférieur, un radical aralcoyle inférieur ou alcényle inférieur, ou bien $R_2$ et $R_3$, ensemble, forment une chaîne alcoylène ayant de 2 à 6 atomes de carbone, éventuellement interrompue par 1 ou 2 hétéroatomes

BAD ORIGINAL

choisis dans le groupe constitué par un oxygène, un soufre et le radical N-$R_6$ ($R_6$ est de l'hydrogène, un radical alcoyle inférieur, un radical hydroxyalcoyle inférieur, un radical alcoxyalcoyle inférieur ou acyloxyalcoyle inférieur),

$R_4$ et $R_5$, distinctement l'un de l'autre, sont de l'hydrogène, un radical alcoyle inférieur, un halogène, un radical trifluorométhyle, un radical alcoxy inférieur, un groupement alcoylènedioxy, un hydroxy, un radical thio, un radical alcoyl thio inférieur, un radical trichlorométhoxy, un radical trifluorométhoxy, un radical trifluorométhyl thio, un radical amino, un radical alcoylamino inférieur, un radical arylamino, un radical alcoylamino sulfonyle inférieur, un radical morpholino sulfonyle, un radical aminosulfonyle, un cyano, un nitro, un carboxy, un radical alcoyloxycarbonyl, un groupe carbonamido, un sulfinyle, un sulfonyle, un radical formyle ou un radical acyle inférieur,

$R_7$ est un radical alcoyle inférieur, un radical phényle ou un radical phényle éventuellement substitué par un radical $R_4$,

m et m', distinctement l'un de l'autre, varient de 1 à 3.

Pour autant que l'invention est concernée, un radical alcoyle inférieur représente une chaîne hydrocarbonée ayant de 1 à 6, de préférence 1 à 3 atomes de carbone, en chaîne droite ou ramifiée; un radical alcényle inférieur représente une chaîne éthylénique ayant 2 à 6, de préférence 2 à 4, atomes de carbone, éventuellement substituée par des radicaux méthyle ; un radical aralcoyle inférieur représente un noyau phényle ou phényle substitué par 1, 2 ou 3 substituants, porteur d'une chaîne alcoyle définie comme ci-dessus. D'une façon générale, l'indication "inférieur" vise à préciser, losrqu'elle est utilisée en combinaison avec tout autre groupe contenant une partie alcoyle, que celle-ci ne comporte pas plus de 6 atomes de carbone.

L'invention se rapporte également aux sels des composés de formule générale I avec un acide minéral ou organique, de préférence thérapeutiquement compatible. Elle s'étend naturellement aussi aux antipodes optiques lorsque ceux-ci existent.

Une classe préférée des composés selon l'invention est définie par la formule générale I dans laquelle A et B ont les signifi-

BAD ORIGINAL

cations sus-indiquées ; $R_4$ et $R_5$ sont de l'hydrogène ou représentent 1, 2 ou 3 substituants choisis parmi les alcoyle inférieur, halogénure, trifluorométhyle, alcoxy inférieur, amino et alcoyloxycarbonyle ; X est de l'oxygène, un groupe N-alcoyle inférieur ou N-phényle ou N-phényle substitué par 1, 2 ou 3 substituants, tels que ceux définis en rapport avec $R_4$ et $R_5$ ; $R_7$ est un radical alcoyle inférieur, un radical phényle ou un radical phényle substitué par 1, 2 ou 3 substituants choisis parmi ceux qui ont été définis à propos de $R_4$ et $R_5$, et le substituant à l'azote azépinique est un groupe

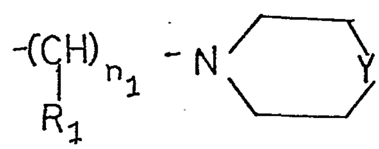

dans lequel $n_1$ vaut 1, 2 ou 3, $R_1$ étant de l'hydrogène ou un radical méthyle ; Y est un groupe $N-R_6$ ou de l'oxygène et $R_6$ est de l'hydrogène, un radical alcoyle inférieur, un radical hydroxy - alcoyle inférieur, un radical alcoxy-alcoyle inférieur ou acyloxyalcoyle inférieur.

Parmi les sous-classes préférées de composés selon l'invention, on mentionnera notamment :

- celle caractérisée par la formule générale I dans laquelle A et B ont les significations sus-indiquées : X est de l'oxygène, un groupe N-alcoyle inférieur ou $N-C_6H_5$, $R_4$ et $R_5$ sont de l'hydrogène : $R_7$ est de l'hydrogène ou un groupe phényle, le cas échéant substitué par un halogène,
- celle caractérisée par la formule générale I dans laquelle A et B ont les significations sus-indiquées : X est de l'oxygène, un groupe N-alcoyle inférieur ou $N-C_6H_5$, $R_4$ et $R_5$ sont de l'hydrogène ; $R_7$ est de l'hydrogène ou un groupe phényle, le cas échéant substitué par un halogène, n varie de 2 à 5 avec au plus un seul des radicaux $R_1$ est un méthyle, les autres $R_1$ étant de l'hydrogène et $R_2$ et $R_3$ sont des groupes méthyle ou éthyle.
- celle caractérisée par la formule générale I dans laquelle A et B ont

les significations sus-indiquées ; X est de l'oxygène, un groupe N-alcoyle inférieur ou $N-C_6H_5$ ; $R_4$ et $R_5$ sont de l'hydrogène ; $R_7$ est de l'hydrogène ou un groupe phényle, le cas échéant substitué par un halogène ; le substituant à l'azote azépinique est un radical

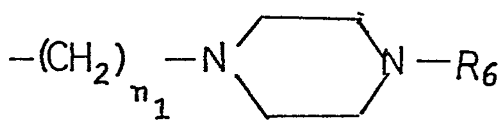

dans lequel $n_1$ vaut 1, 2 ou 3 et $R_6$ a la signification sus-indiquée, notamment hydroxyméthyle ou hydroxyéthyle.

Parmi les composés de formule générale I, on pourra distinguer plus particulièrement les sous-groupes suivants :

a) les isoxazolo [3,4-d] -dibenzazépines de formule générale $I_a$ dans laquelle le groupe X représente de l'oxygène. Ils répondent à la formule générale $I_a$

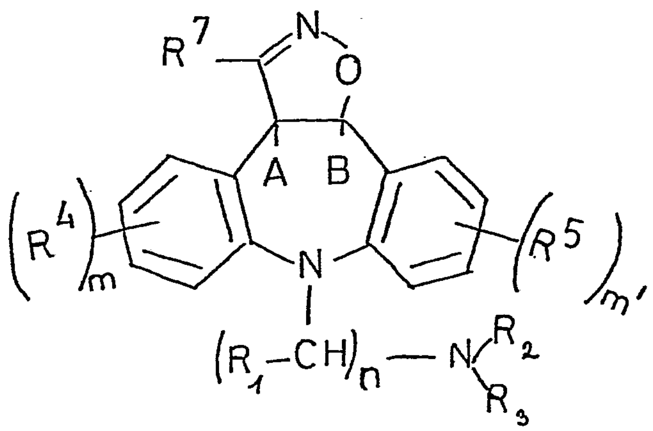

(Ia)

dans laquelle la définition des substituants R, $R_1$, $R_2$, $R_3$, $R_4$, $R_5$, $R_7$, n, m et m' demeure celle fournie précédemment et A et B représentent chacun un atome d'hydrogène.

b) les pyrazolo [4,5-d] -dibenzazépines de formule générale $I_b$ dans laquelle X représente le groupe $N-R_8$. Ils répondent à la formule générale $I_b$

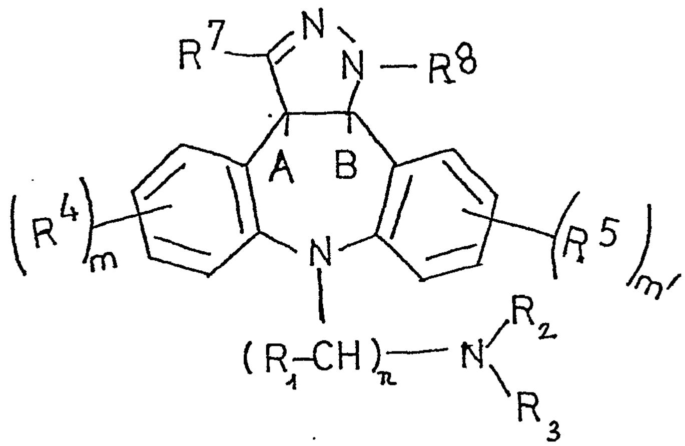

(Ib)

dans laquelle la définition des substituants $R_1$, $R_2$, $R_3$, $R_4$, $R_5$, $R_7$, n, m et m' demeure inchangée et $R_8$ représente un radical phényle, un radical phényle substitué par 1, 2 ou 3 substituants ou un radical alcoyle inférieur et A et B représentent chacun un atome d'hydrogène.

c) les oxazolo [3,4-d] -dibenzazépines de formule générale $I_c$ dans laquelle X représente de l'oxygène et A et B forment ensemble une double liaison C = C. Ils sont représentés par la formule générale $I_c$

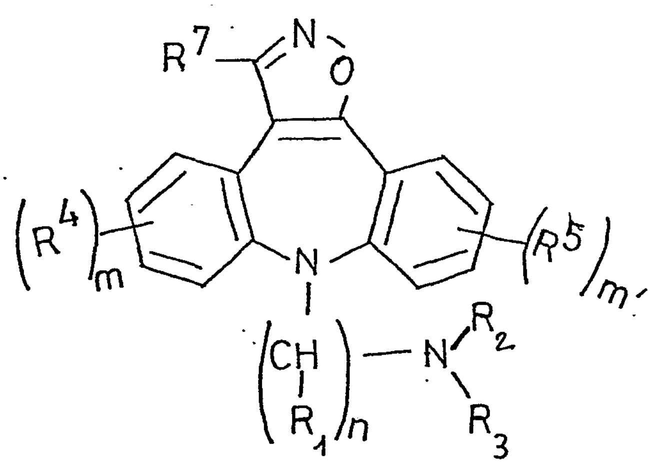

(Ic)

dans laquelle les substituants $R_1$, $R_2$, $R_3$, $R_4$, $R_5$, $R_7$, n, m et m' sont définis comme précédemment.

d) les pyrazolo [4,5-d] -dibenzazépines de formule générale $I_d$ dans laquelle X représente un groupe N-$R_8$ et A et B forment ensemble une double liaison C = C. Ils sont représentés par la formule générale $I_d$ :

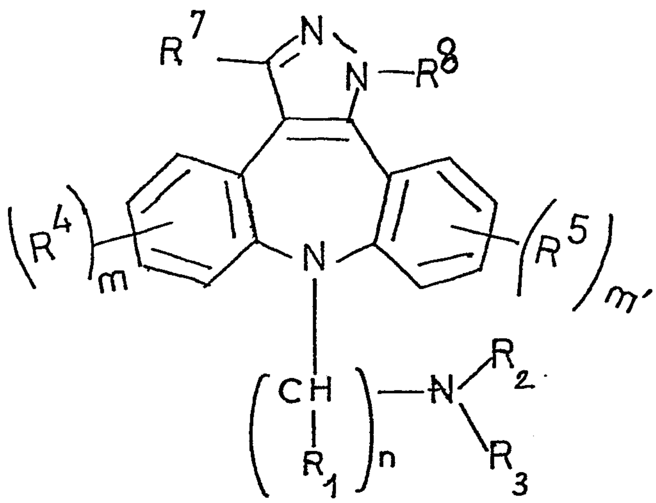

(Id)

dans laquelle les substituants $R_1$, $R_2$, $R_3$, $R_4$, $R_5$, $R_7$, $R_8$, n, m et m' sont définis comme précédemment.

Lorsque $R_2$ et $R_3$ forment ensemble une chaîne alcoylène, ils forment avec l'atome d'azote adjacent un hétérocycle azoté comme par exemple l'azétidine, la pyrrolidine, la pipéridine, l'hexaméthylène imine, l'heptaméthylène imine.

Lorsque la chaîne alcoylène est interrompue par 1 ou 2 hétéroatomes, on pourra avoir un cycle oxazolidine, iso oxazolidine, thiazolidine, morpholine, thiomorpholine ou homomorpholine.

Lorsque la chaîne alcoylène est interrompue par un groupe azoté, on pourra avoir un radical pyrazolidine, pipérazine, homopipérazine, et les dérivés N-alcoylés de ceux-ci.

Parmi les sels d'addition avec un acide minéral ou organique thérapeutiquement compatible, on citera à titre d'exemples le

chlorhydrate, le bromhydrate, les sulfates, les phosphates, le méthane sulfonate, l'acétate, le fumarate, le succinate, le lactate, le pyruvate, le citrate, le tartrate, le maléate, le malonate, le cétogulonate, le benzoate, le salicylate, le dichloro-2,6 benzoate, le triméthoxy benzoate, le diaminobenzène sulfonate, le chromoglycate, le benzène sulfonate, le dipropyl acétate, le glucose-1 phosphate ou le pamoate. Les sels peu solubles peuvent être intéressants pour la réalisation de préparations médicamenteuses à effet retard ou encore pour la réalisation de purifications par cristallisations.

Les sels d'addition avec un acide minéral ou organique thérapeutiquement non compatible font également partie de l'invention comme moyen d'identification, de purification, de séparation ou de dédoublement. On pourra citer en particulier le perchlorate, le periodate, le d-camphorate, le N,N-diéthyl d-tartramate, le ditoluyl d-tartrate ou l'itaconate.

L'invention comprend aussi un procédé d'obtention des composés hydrogénés de formule $I_a$ et $I_b$

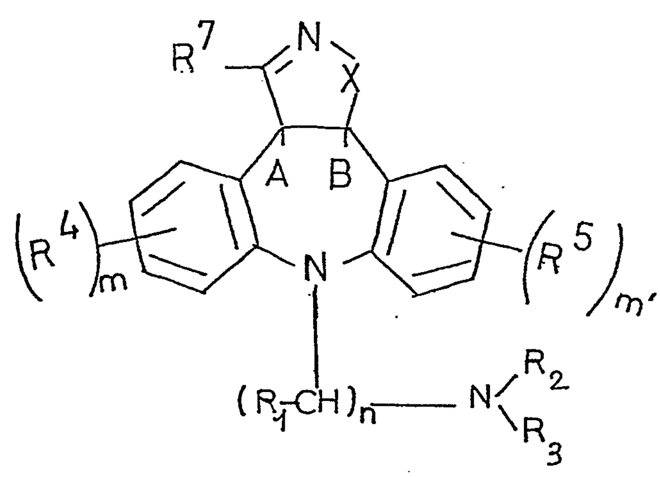

dans laquelle les substituants $R_1$, $R_2$, $R_3$, $R_4$, $R_5$, $R_7$, n, m et m' sont définis comme précédemment, et X représente de l'oxygène ou le radical $N-R_8$ (dans lequel $R_8$ garde les significations fournies antérieurement),

caractérisé en ce que l'on fait agir au sein d'un solvant inerte et non polaire, tel que le benzène ou le toluène, de préférence à reflux, un sel de métal alcalin d'un imino stilbène de formule générale (II)

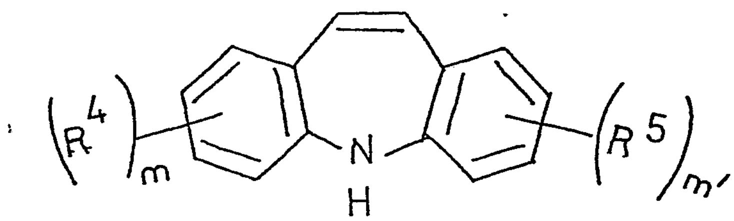

( II )

dans laquelle $R_4$, $R_5$, m et m' ont les significations fournies antérieurement, avec un halogénure d'amino alcoylène de formule générale ( III ) dans laquelle Hal représente du chlore ou du brome

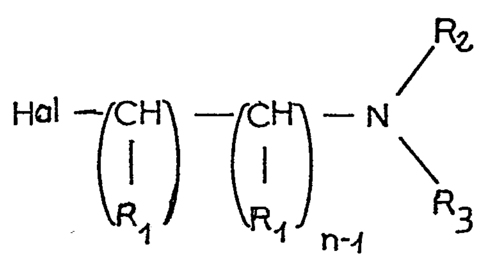

( III )

pour obtenir la N-alcoylamino alcoyl dibenzazépine de formule générale (IV)

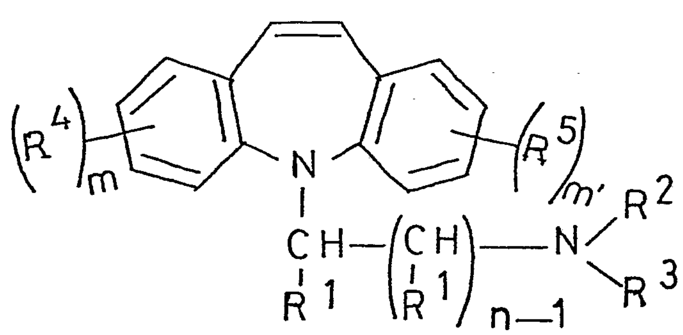

( IV )

dans laquelle les substituants $R_1$, $R_2$, $R_3$, $R_4$, $R_5$, m, m' et n sont définis comme précédemment, puis on effectue, au sein d'un solvant inerte, non polaire, tel que le benzène ou le toluène, une réaction de condensation dipolaire 1-3 avec un oxyde de nitrile ou avec une nitrile imine de formule :

$$R_7 - \overset{\oplus}{C} = N - \overset{\ominus}{N} - R_8$$

ou

$$R_7 - \overset{\oplus}{C} = N - \overset{\ominus}{O}$$

dans laquelle les substituants $R_7$ et $R_8$ sont définis comme précédemment, en milieu basique à reflux du solvant, pour obtenir l'isoxazolo- ou la pyrazolo- dibenzazépine désirée de formule $I_a$ ou $I_b$ que l'on peut si désiré salifier par addition d'un acide minéral ou organique, ou dédoubler par salification par un acide organique optiquement-actif ou déshydrogéner en composé $I_c$ ou $I_d$ par chauffage avec un agent de déshydrogénation ou dans un solvant neutre et à point d'ébullition élevé.

Selon un mode particulier d'obtention des isoxazolo-[4,5-d]-dibenzazépines de formule générale $I_a$, on effectue la réaction de condensation dipolaire en générant <u>in situ</u> et à température ordinaire le nitrile-oxyde par réaction de la triéthylamine avec une α-chloroaldoxime en solution dans un solvant inerte, comme le benzène ou le toluène, puis en le condensant au sein du même solvant, avec l'(amino alcoyl) dibenzazépine de formule générale IV.

Les pyrazolo [3,4-d]-dibenzazépines sont préparées selon la méthode décrite par HUISGEN et coll. dans Tetrahedron (1962), 17, 3, pour la diphényl-1,3 pyrazolo [3,4-d] dibenzo [b,f] azépine 6 seul composé déjà décrit, mais sur le seul plan de la chimie, en générant "in situ" une nitrile imine à partir d'un hydrazono chlorure dans un solvant inerte, tel que le benzène ou le toluène, puis chauffage à reflux pendant 16 heures dans le même solvant avec l'imino stilbène en présence de triéthylamine.

Selon l'invention, les composés déshydrogénés de formule générale $I_c$ et $I_d$ peuvent également être obtenus en condensant sur un imino stilbène de formule générale (II)

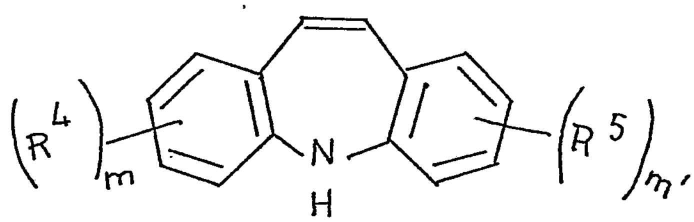

(II)

dans laquelle les substituants $R_4$, $R_5$, m et m' sont définis comme précédemment, selon la réaction d'addition dipolaire 1-3, un N-oxyde de nitrile de formule $R_7 - \overset{\oplus}{C} = N - \overset{\ominus}{O}$ ou une nitrile - imine de formule

$$R_7 - \overset{\oplus}{C} = N - \overset{\ominus}{N} - R_8$$

pour obtenir une isoxazolo [4,5-d]- ou une pyrazolo[3,4-d]-dibenzazépine de formule générale V

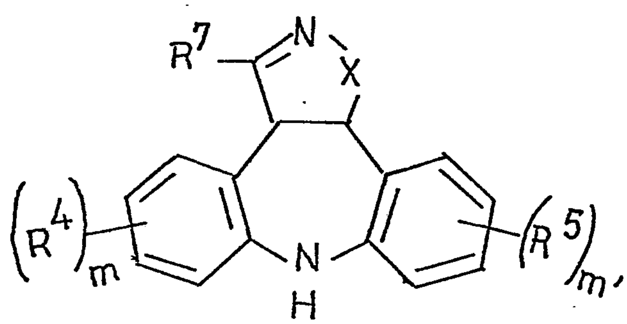

(V)

dans laquelle X représente de l'oxygène ou le radical $N-R_8$ et dans laquelle les substituants $R_4$, $R_5$, $R_7$, m et m' sont définis comme précédemment, puis soumet ce composé à une déshydrogénation, au moyen d'un agent de déshydrogénation tel que le chloranile dans le tétrahydrofuranne, ou par chauffage dans un solvant neutre à point d'ébullition élevé tel que le nitrobenzène ou le phénétole, pour obtenir l'isoxazole ou le pyrazole correspondant de formule générale VI

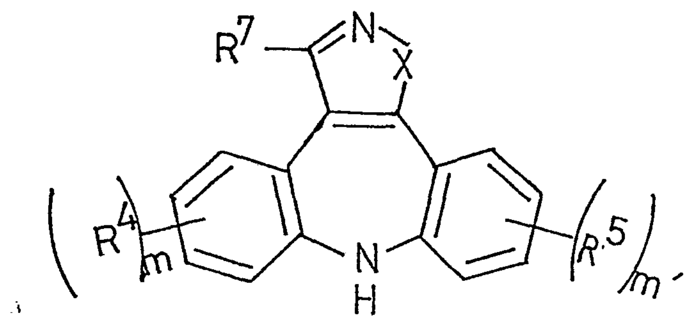

(VI)

dans laquelle les substituants $R_4$, $R_5$, $R_7$ et X ont les significations fournies précédemment, que l'on fait réagir avec un agent de métallation, tel qu'un amidure alcalin, notamment l'amidure de sodium, dans un solvant inerte tel que le benzène ou le toluène, pour fournir le dérivé métallique correspondant, puis condense celui-ci avec un halogénure d'aminoalcoylène de formule générale III

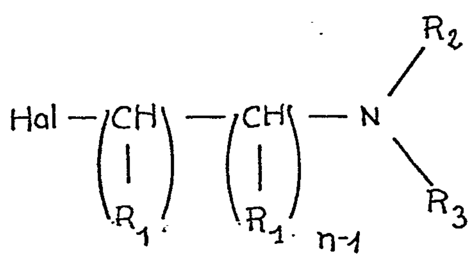

( III )

dans laquelle les substituants $R_1$, $R_2$, $R_3$ et n sont définis comme précédemment, pour former le composé de formule générale $I_c$ ou $I_d$ désiré que l'on peut si nécessaire salifier ou dédoubler lorsque la molécule comporte un atome de carbone chiral.

Selon une autre variante du procédé au départ des composés de formule générale V, on soumet le composé à une métallation par un dérivé de métal alcalin, tel qu'un amidure, notamment de sodium, dans le susdit solvant inerte, puis fait réagir, au sein du même solvant le dérivé métallique obtenu avec un halogénure d'aminoalcoylène de formule générale III, dans laquelle Hal est du brome ou du chlore, pour obtenir un

composé de formule générale VII

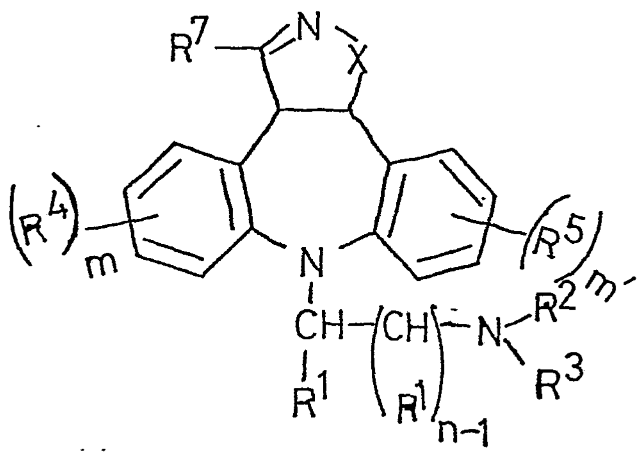

(VII)

que l'on déshydrogène par action d'un agent de déshydrogénation, tel le chloranile dans le tétrahydrofuranne, ou par chauffage dans un solvant neutre à point d'ébullition élevé, comme le nitrobenzène ou le phénétole.

Dans tous les cas les dérivés de formule générale I sont cristallisés. Ils ont été identifiés par leur point de fusion, leur Rf en chromatographie sur couche mince de silice, ainsi que par leur microanalyse, et pour les bases correspondantes par les spectres de $^{1}H$ et de $^{13}C$ RMN.

La cycloaddition dipolaire 1-3 est une réaction d'addition cis de telle sorte que la stéréochimie à la jonction des cycles isoxazolo ou pyrazolo et dibenzazépine est fixée. Ainsi un seul composé racémique est obtenu pour les composés de formule générale $I_a$ et $I_b$, pour autant que la chaîne aminoalcoylène (du groupe R) ne comporte pas de carbone chiral et que les deux cycles aromatiques ne sont pas substitués. Ce racémique est dédoublable en ses deux antipodes optiques par salification à l'aide d'un acide organique optiquement actif.

Dans les composés de formule $I_a$ ou $I_b$, la jonction cis au niveau des deux dihétérocycles se vérifie bien par les constantes de couplage des protons portés par les carbones de jonction.

Selon un mode d'exécution préféré du procédé selon l'invention, la réaction de déshydrogénation est effectuée au moyen du nitrobenzène entre 120°C et le point d'ébullition, ou avec le chloranile au reflux du tétrahydrofuranne.

Parmi les solvants à haut point d'ébullition on pourra utiliser le phénétole, la décaline, la tétraline.

Les dérivés ainsi préparés sont obtenus à l'état d'halogénohydrates, notamment de chlorhydrates ou de bromhydrates.

De façon en soi classique, on peut obtenir les bases en les faisant réagir en solution avec un hydroxyde de préférence alcalin, tel que la soude et la potasse.

Par salification de la base obtenue avec l'acide choisi, dans des conditions également classiques, on peut passer aux sels correspondants des dérivés en question.

L'invention se rapporte aussi aux compositions pharmaceutiques notamment utiles en tant qu'antidépresseurs pour le traitement des états dépressifs de toute nature, en tant qu'analgésiques et médicaments spasmolytiques, renfermant à titre de principe actif au moins un composé de formule I, dans laquelle les groupes A, B, X, $R_2$,$R_3$, $R_4$,$R_5$, et $R_7$ ont les significations sus-indiquées ainsi que le composé de formule I dans laquelle, simultanément, A, B,$R_4$ et $R_5$ représentent de l'hydrogène, $R_7$ est un groupe phényle et X représente le groupe N-phényle, ledit principe se trouvant, le cas échéant, sous forme racémique ou dédoublée, ou sous la forme d'un de ses sels d'addition avec un acide minéral ou organique, en association ou en mélange avec un excipient ou un véhicule inerte non toxique, pharmaceutiquement acceptable.

Selon l'invention, le véhicule ou l'excipient inerte sont ceux qui conviennent pour l'administration par voie parentérale, buccale, rectale, perlinguale ou percutanée.

On pourra citer parmi ces compositions pharmaceutiques les comprimés, les comprimés enrobés, les dragées, les tablettes, les gélules, les capsules, les solutions ou émulsions buvables, les gouttes, les sirops, les solutés ou suspensions injectables conditionnées en ampoules, en flacons multidoses ou en seringues auto-injectables ; les suppositoires, les comprimés sublinguaux, les solutions dans un solvant po-

laire pour application percutanée.

La posologie unitaire est variable selon la voie d'administration. Elle s'échelonne entre 25 et 150 mg par prise unitaire, de préférence entre 50 et 100 mg par prise. La posologie journalière chez l'adulte s'échelonne entre 50 et 600 mg.

Les composés selon l'invention sont des antidépresseurs utiles pour le traitement des états dépressifs de toute nature (dépressions réactionnelles, dépressions endogènes, dépressions psychotiques de type mélancolique...). Ils sont également doués de propriétés analgésiques importantes et de propriétés spasmolytiques.

D'autres caractéristiques de l'invention apparaîtront encore au cours de la description qui suit d'exemples de composés préférés dont seront indiqués les modes de préparation, les caractéristiques physicochimiques et les propriétés biologiques.

Les nombres soulignés d'un double trait, qui suivent immédiatement le nom des composés identifiés dans les exemples, serviront à les désigner dans les essais biologiques qui suivront. On a également fait figurer immédiatement après ce nombre et dans certains des exemples la catégorie dans laquelle se range l'exemple considéré, par référence aux formules indiquées ci-dessus Ia, Ib, Ic ou Id.

Exemple I

phényl-3 trihydro-3a,8,12b (dibenzo[b,f] isoxazolo [4,5-d])azépine
(1, Ia, $R = R_4 = R_5 = H, R_7 = C_6H_5$)

On dissout dans 500 ml de benzène et 200 ml d'éther anhydre 20 g d'α-chlorobenzaldoxime puis 24 g d'iminostilbène. On agite le mélange sous atmosphère inerte puis on ajoute goutte à goutte 100 ml de triéthylamine dans 100 ml de benzène anhydre. L'addition dure environ 6 h, puis le mélange réactionnel est chauffé pendant 1 h au reflux. Le chlorhydrate de triéthylamine précipite progressivement. On le sépare par filtration. Le filtrat est évaporé à siccité sous pression réduite.

On obtient 17g d'un mélange de produit de départ et de dibenzo isoxazolo azépine.

7 g de ce mélange sont dissous dans 200 ml de chloroforme puis la solution est passée sur une colonne de chromatographie, chargée par 180 g de gel de silice dans le chloroforme. Après fixation, la colonne est éluée au chloroforme. On sépare d'abord l'iminostilbène (zone orange, 8 fractions de 40 ml), puis le produit de condensation (8 fractions de 100 ml). Par évaporation on recueille 2,5 g de produit soit 35% ; F > 260°C.

Analyse $C_{21}H_{16}N_2O = 312$

| | C | H | N% |
|---|---|---|---|
| Calculé | 30,84 | 5,17 | 8,98 |
| Trouvé | 30,51 | 5,05 | 9,22 |

En CCM sur plaque de silice, Rf = 0,3 (solvant d'élution: benzène 70, chlorure de méthylène 20, méthanol 10); révélation U.V. : fluorescence jaune.

Selon le même mode opératoire, les dérivés N-substitués suivants ont été obtenus à partir des iminostilbènes N-aminoalkylés.

1°) (β-diméthylaminoéthyl)-8 phényl- 3 trihydro-3a,8,12b (dibenzo [b,f] isoxazolo [4,5-d])azépine (2, Ia, $R = (CH_2)_2-N(CH_3)_2$, $R_4 = R_5 = H$, $R_7 = C_6H_5$) isolée sous forme de chlorhydrate Rdt = 46 %
F > 260°C

Analyse $C_{25}H_{26}N_3O\ Cl = 419,5$

| | C | H | N% |
|---|---|---|---|
| Calculé | 71,15 | 6,21 | 9,96 |
| Trouvé | 71,25 | 6,36 | 9,98 |

En CCM, Rf = 0,5 (solvant d'élution $CHCl_3$ + 15% méthanol) ; révélation par la berbérine.

Préparation du méthanesulfonate : à la base dissoute dans un minimum d'éthanol, on ajoute une quantité stoechiométrique d'acide

méthane sulfonique. Le sel cristallise après une nuit au réfrigérateur. Il est recristallisé dans le minimum d'éthanol ; rendement pratiquement quantitatif, F = 235° C.

Analyse $C_{26}H_{29}N_3O_4S$ = 479,5

| | C | H | N | S% |
|---|---|---|---|---|
| Calculé | 65,12 | 6,10 | 8,77 | 6,69 |
| Trouvé | 65,16 | 6,07 | 8,66 | 6,65 |

2°) (γ-diméthylaminopropyl)-8 phényl-3 trihydro-3a, 8,12b (dibenzo [b,f ] isoxazolo [4,5-d])azépine ($\underline{\underline{3}}$, Ia, R = $(CH_2)_3$-$N(CH_3)_2$,$R_4$ = $R_5$ = H, $R_7$ = $C_6H_5$), isolée sous forme de chlorhydrate hydraté F = 180° C, Rdt = 50%.

Analyse $C_{26}H_{28}N_3O$ Cl, $1H_2O$ = 452

| | C | H | N% |
|---|---|---|---|
| Calculé | 69,08 | 6,69 | 9,30 |
| Trouvé | 68,68 | 6,67 | 9,22 |

En CCM, Rf = 0,38 (solvant : chloroforme + 15% méthanol) révélation par la berbérine.

3°) (méthyl-2 diméthylamino-3 propyl)-8 phényl-3 trihydro-3a, 8, 12b (dibenzo[b,f ]-isoxazolo [4,5-d]) azépine ($\underline{\underline{4}}$, Ia, R = $CH_2$-$CH(CH_3)$-$CH_2$-$N(CH_3)_2$, $R_4$=$R_5$=H, $R_7$=$C_6H_5$), isolée sous forme de chlorhydrate Rdt = 43%

F $>$ 260°C

Analyse $C_{27}H_{30}N_3O$ Cl, $0,5H_2O$ = 457

| | C | H | N% |
|---|---|---|---|
| Calculé | 70,96 | 6,84 | 9,19 |
| Trouvé | 71,27 | 6,82 | 9,11 |

En CCM, Rf = 0,49 (solvant : $CHCl_3$ + 15 % méthanol); révélation par la berbérine.

4°) [1'-(β-hydroxyéthyl-4') pipérazinyl]-3 propyl -8 phényl-3 trihydro-3a, 8, 12b(dibenzo[b,f]isoxazolo [4,5-d]-azépine (5, Ia, $R_4$=$R_5$=H, $R_7$=$C_6H_5$), R = $(CH_2)_3$-N 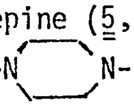 N-$(CH_2)_2$OH) isolée sous forme de chlorhydrate,F=195°C

Rdt= 54 %

Analyse $C_{30}H_{36}N_4O_2Cl_2$, 1,5 $H_2O$ = 582,5

| | C | H | N% |
|---|---|---|---|
| Calculé | 61,85 | 6,74 | 9,61 |
| Trouvé | 61,87 | 7,15 | 9,30 |

En CCM, Rf = 0,62 (solvant d'élution $CH_2Cl_2$ + 5 % éthanol) révélation par U.V. : fluorescence bleue.

Exemple II

(γ-diméthylaminopropyl)-8 phényl-3 trihydro-3a, 8, 12b (dibenzo[b,f]isoxazolo[4,5-d])azépine (3, Ia, R = $CH_2$-$CH(CH_3)$ - $CH_2$ - $N(CH_3)_2$ ; $R_4$=$R_5$=H ; $R_7$ = $C_6H_5$).

Dans 200 ml de benzène, on dissout 27,8 g de (γ-diméthyl-aminopropyl)-5 dibenzo[b,f] 5H azépine et 15,5 g d'α-chlorobenzaldoxime. Sous courant d'azote, on ajoute ensuite très progressivement 20 ml de triéthylamine dissous dans 50 ml de benzène. On maintient l'agitation pendant 20 heures, puis on chauffe une heure au reflux. On essore le précipité de chlorhydrate de triéthylamine. Le filtrat est alors évaporé à sec sous pression réduite. Le résidu huileux obtenu est dissous dans 100 ml d'isopropanol et on ajoute de l'acide chlorhydrique 12N jusqu'à ce que la solution présente une réaction acide. Le chlorhydrate de la dibenzo isoxazolo azépine cristallise par grattage. On recristallise dans l'isopropanol et on obtient 20 g de sel, soit 50% de rendement ; F = 180° C.

Exemple III

(γ-diméthylaminopropyl)-8 diphényl-1,3 tétrahydro-1, 3a,8, 12b(dibenzo [b,f]pyrazolo [3,4-d])azépine (8, Ib, R = $(CH_2)_3-N(CH_3)_2$ ; $R_4=R_5=H$ ; $R_7=R_8=C_6H_5$).

Stade A

5-(γ-diméthylaminopropyl)- dibenzo[b,f] 5 H azépine.

On met en suspension 96,5 g d'iminostilbène dans 2 litres de toluène sec. On ajoute 80g d'une suspension d'amidure de sodium dans le toluène sous forme d'une pâte à 50% et on agite à reflux du to-luène pendant 8 heures. On ajoute à cette solution 53,5 g de chlorure de diméthylamino-2 éthyle fraîchement libéré de son chlorhydrate (par l'ammoniaque, puis extraction au toluène), et on porte au reflux pendant 16 heures sous agitation.

Après refroidissement à température ordinaire, on ajoute 2 litres de glace, puis on décante la phase toluénique. On évapore le solvant sous pression réduite. L'huile obtenue est distillée sous vide à 160° C sous 2 mm. Il passe par distillation 120 g d'un produit huileux épais qui cristallise progressivement. Le rendement en produit pur est de 90%.

Stade B

(γ-diméthylaminopropyl)-8 diphényl-1,3 tétrahydro-1, 3a, 8,12b (dibenzo [b,f] pyrazolo [3,4-d]) azépine, 8.

On dissout 27,8 g de la dibenzazépine du stade A et 23g de N-α-chlorobenzylidène phénylhydrazine dans 200 ml de benzène sec. On porte le mélange au reflux sous courant d'azote et on ajoute goutte à goutte une solution de 20 ml de triéthylamine dans 50 ml de benzène sec tout en maintenant l'agitation. On poursuit le chauffage au reflux pendant 24 heures. Le chlorhydrate de triéthylamine précipite quantitativement. On le sépare par filtration, on l'essore et on le rince. Les filtrats sont réunis et évaporés à sec sous pression réduite. On obtient ainsi 47 g d'un produit huileux qui cristallise. On le recristallise dans l'isopropanol pour obtenir 31 g de produit pur, soit un rendement de 65%.

Ce produit fond à 170°C. Il est converti en chlorhydrate qui fond à 246°C.

Analyse $C_{32}H_{33}N_4Cl$, 1,5 $H_2O$ = 536

| | C | H | N% |
|---|---|---|---|
| Calculé | 71,70 | 6,77 | 10,43 |
| Trouvé | 71,91 | 7,04 | 10,17 |

En CCM, Rf = 0,49 (solvant élution : chloroforme + 15% méthanol) ; révélation par U.V. : fluorescence bleue. Les dibenzo [b,f] pyrazolidino [3,4-d] azépines suivantes ont été obtenues selon le même mode opératoire.

a) Le (β-diméthylaminoéthyl)-8 diphényl-1,3 tétrahydro-1, 3a, 8, 12b (dibenzo [b,f] pyrazolo [3,4-d]) azépine (7, Ib, R = $(CH_2)_2-N(CH_3)_2$ ; $R_4=R_5=H$ ; $R_7=R_8=C_6H_5$) sous forme de chlorhydrate, F > 260°C ; Rdt = 34%.

Analyse $C_{31}H_{31}N_4Cl$, 1 $H_2O$ = 513

| | C | H | N% |
|---|---|---|---|
| Calculé | 72,58 | 6,48 | 10,92 |
| Trouvé | 72,33 | 6,63 | 10,79 |

En CCM, Rf = 0,46 (solvant : chloroforme + 15% méthanol) ; révélation par U.V. : fluorescence bleue.

b) Le (méthyl-2 diméthylamino-3 propyl)-8 diphényl-1,3 tétrahydro-1, 3a, 8, 12b (dibenzo [b,f] pyrazolo [3,4-d])azépine (9, Ib, R = $CH_2-CH(CH_3)-CH_2-N(CH_3)_2$; $R_4=R_5=H$ ; $R_7=R_8=C_6H_5$) sous forme de chlorhydrate F >260°C ; Rdt = 30,5 %.

Analyse $C_{33}H_{35}N_4Cl$, 1,5 $H_2O$ = 550

| | C | H | N% |
|---|---|---|---|
| Calculé | 72,06 | 6,96 | 10,19 |
| Trouvé | 72,29 | 6,76 | 10,15 |

En CCM, Rf = 0,63 (solvant : chloroforme + 15% méthanol) ; révélation par U.V. : fluorescence bleue.

c) Le ( [1'-(β-hydroxyéthyl-4') pipérazinyl] -3propyl)-8 diphényl-1,3 tétrahydro-1,3a, 8,12b(dibenzo[b,f] pyrazolo [3,4-d])azépine (10, Ib, $R_4$=$R_5$=H ; $R_7$=$R_8$=$C_6H_5$; R = $(CH_2)_3$- N 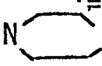 N-$(CH_2)_2$OH) isolé sous forme de chlorhydrate, F = 175° C, Rdt = 31%.

<u>Analyse</u> $C_{36}H_{41}N_5O_2Cl_2$, 1,5 $H_2O$ = 658

| | C | H | N% |
|---|---|---|---|
| Calculé | 65,76 | 6,75 | 10,65 |
| Trouvé | 65,49 | 6,82 | 10,82 |

En CCM, Rf = 0,59 (solvant d'élution : $CH_2Cl_2$ + 5% éthanol), révélation en U.V. : fluorescence bleue.

d) la diphényl-1,3 tétrahydro-1,3a,8,12b(dibenzo [b,f]pyrazolo[3,4-d])azépine (6, Ib, $R_4$=$R_5$=H ; $R_7$=$R_8$=$C_6H_5$), fondant au-dessus de 260°C, Rdt = 51%.

<u>Analyse</u> $C_{27}H_{21}N_3$ = 387

| | C | H | N% |
|---|---|---|---|
| Calculé | 83,79 | 5,47 | 10,86 |
| Trouvé | 83,57 | 5,45 | 10,94 |

En CCM, Rf = 0,2 (solvant : benzène 70%, $CH_2Cl_2$ 20%, méthanol 10%), révélation par U.V. : fluorescence bleue.

e) Le (γ-diméthylaminopropyl)-8 phényl-1 (chloro-4' phényl)-3 tétrahydro-1,3a, 8,12b(dibenzo[b,f]pyrazolo [3,4-d])azépine (11, Ib, $R_1$= $(CH_2)_3$-$N(CH_3)_2$; $R_4$=$R_5$=H ; $R_7$ = $C_6H_4$-$Cl_{(p)}$, $R_8$=$C_6H_5$) sous forme de chlorhydrate, F = 215°C, Rdt = 76%.

<u>Analyse</u> $C_{31}H_{32}N_4Cl_2$, 1 $H_2O$ = 561

| | C | H | N% |
|---|---|---|---|
| Calculé | 68,44 | 6,06 | 9,98 |
| Trouvé | 68,35 | 6,15 | 9,83 |

En CCM, Rf = 0,1 ($CHCl_3$ + 15% méthanol), révélation par U.V. : fluorescence bleue.

Exemple IV

diphényl-1,3 dihydro-1,8 (dibenzo[b,f]pyrazolo[3,4-d]) azépine ($\underline{\underline{12}}$, Id, $R_4$=$R_5$=H ; $R_7$=$R_8$=$C_6H_5$).

5 g de diphényl-1,3 (dibenzo[b,f] pyrazolo[3,4-d]) 5 H azépine $\underline{\underline{6}}$ sont dissous dans 500 ml de nitrobenzène. On chauffe la solution à 150°-155°C pendant 36 heures, puis on évapore le nitrobenzène sous pression réduite. Le résidu cristallise. On le sépare par essorage, on le lave à l'éther et on le purifie par recristallisation de l'éthanol pour obtenir 3,4 g de pyrazole, soit un rendement de 68%. Le produit fond à 252°C.

Analyse $C_{27}H_{19}N_3$ = 385,5

| | C | H | N% |
|---|---|---|---|
| Calculé | 84,10 | 4,96 | 10,92 |
| Trouvé | 84,23 | 4,76 | 10,94 |

En CCM, Rf = 0,7 ($CH_2Cl_2$ + 5% méthanol) ; révélation par U.V. : fluorescence jaune.

La réaction de déshydrogénation peut également être effectuée en solution dans le tétrahydrofuranne par addition de chloranile (2 g de $\underline{\underline{6}}$, 2 g de chloranile, 250 ml de THF). On porte à l'ébullition pendant 24 heures. Après évaporation du solvant, on reprend le résidu sec par de la lessive de potasse. La phase aqueuse est épuisée au chloroforme. On évapore ensuite le chloroforme. Le pyrazole cristallise quantitativement. On le recristallise de l'éthanol.

Les constantes physiques sont identiques à celles du produit obtenu par déshydrogénation par le nitrobenzène.

Exemple V

($\gamma$-diméthylaminopropyl)-8 phényl-3 (dibenzo [b,f] isoxazolo [4,5-d] 8H azépine ($\underline{\underline{18}}$, Ic, R = $(CH_2)_3$-$N(CH_3)_2$ ; $R_4$=$R_5$=H ; $R_7$= $C_6H_5$)

2 g de la dibenzo[b,f]isoxazolo [4,5-d] tétrahydro-azépine 16 sont dissous dans 100 ml de toluène sec. On ajoute 1 g d'amidure de sodium et on maintient la suspension sous agitation tout en chauffant 10 heures au reflux du solvant. On ajoute alors une solution de 7 mmoles de chlorure de N,N'-diméthylaminopropyle (libéré de son chlorhydrate par l'ammoniaque et extrait au benzène) dans le benzène et on porte le mélange au reflux pendant 20 heures. Après refroidissement, on ajoute 100 ml d'eau et on épuise la phase benzénique à deux reprises par 25 ml d'acide chlorhydrique à 18%. Les extraits acides sont neutralisés par du carbonate sodique et la base est extraite au chlorure de méthylène. La base cristallise. On la dissout dans un minimum d'isopropanol et on ajoute une solution de gaz chlorhydrique dans l'éther. Le chlorhydrate précipite. On recristallise dans l'isopropanol ;Rdt = 40% ; F = 210°.

Analyse $C_{26}H_{26}N_3OCl$, $1,5H_2O$ = 459

| | C | H | N% |
|---|---|---|---|
| Calculé | 68,03 | 6,38 | 9,16 |
| Trouvé | 67,62 | 6,75 | 8,83 |

En CCM, Rf = 0,27 ($CH_2Cl_2$ + 15 % éthanol) ; révélation par U.V. : fluorescence jaune.

Selon le même procédé, on a obtenu :

1°) La (β-diméthylaminoéthyl)-8 diphényl-1,3 dihydro-1,8 (dibenzo[b,f] pyrazolo[3,4-d] azépine 13, Id;
R =$(CH_2)_2$-$N(CH_3)_2$ ; $R_4$=$R_5$=H ; $R_7$=$R_8$=$C_6H_5$) sous forme de chlorhydrate; Rdt = 30% (sublimation à 250°C).

Analyse $C_{31}H_{29}N_4Cl$, 3 $H_2O$ = 546,5

| | C | H | N% |
|---|---|---|---|
| Calculé | 68,19 | 6,40 | 10,26 |
| Trouvé | 68,58 | 6,56 | 9,95 |

En CCM, Rf = 0,42 ($CH_2Cl_2$ + 15% éthanol) ; révélation par U.V. : fluorescence bleue.

2°) La (méthyl-2 diméthylamino-3 propyl)-8 diphényl 1,3 dihydro-1,8(dibenzo [b,f] pyrazolo [3,4-d]) azépine (15, Id, $R_4$=$R_5$=H,R=$CH_2$-$CH(CH_3)$-$CH_2$-$N(CH_3)_2$ ; $R_7$=$R_8$=$C_6H_5$), sous forme de chlorhydrate, F = 210°C, Rdt = 43%.

Analyse $C_{33}H_{33}N_4Cl$ = 521

| | C | H | N% |
|---|---|---|---|
| Calculé | 76,07 | 6,38 | 10,75 |
| Trouvé | 75,80 | 6,63 | 10,43 |

En CCM, Rf = 0,45 ($CH_2Cl_2$ + 15% éthanol) ; révélation par U.V. : fluorescence bleue.

De la même manière, au départ des dibenzo isoxazolo azépines Ia de l'exemple I, on obtient respectivement :

3°) La phényl-3 (dibenzo [b,f] isoxazolo [4,5-d]) 8H azépine (16, Ic, R=$R_4$=$R_5$=H ; $R_7$=$C_6H_5$), qui fond à 208°C, Rdt = 60%.

Analyse $C_{21}H_{14}N_2O$ = 310

| | C | H | N% |
|---|---|---|---|
| Calculé | 81,36 | 5,55 | 9,04 |
| Trouvé | 81,52 | 5,25 | 8,80 |

En CCM, Rf = 0,85 ($CH_2Cl_2$ + 5 % éthanol) ; révélation par U.V. : fluorescence brune.

4°) La (β-diméthylaminoéthyl)-8 phényl-3 (dibenzo [b,f] isoxazolo [4,5-d]) 8H azépine (17, Ic, R = $(CH_2)_2$-$N(CH_3)_2$; $R_4$=$R_5$=H ; $R_7$=$C_6H_5$).

Analyse $C_{25}H_{24}N_3OCl$ = 418

| | C | H | N% |
|---|---|---|---|
| Calculé | 71,83 | 5,79 | 10,05 |
| Trouvé | 71,34 | 6,20 | 9,75 |

En CCM, Rf = 0,35 ($CH_2Cl_2$ + 15 % éthanol) ; révélation par U.V. : fluorescence jaune.

5°) La (méthyl-2 diméthylamino-3 propyl)-8 phényl-3 (dibenzo [b,f] isoxazolo [3,4-d]) 8H azépine (19, Ic, $R_4=R_5=H$ ; $R_7=C_6H_5$ ; $R=CH_2-CH(CH_3)-CH_2-N(CH_3)_2$), sous forme de chlo-rhydrate, F = 225°C, Rdt = 32%.

Analyse $C_{27}H_{28}N_3OCl$, 1 $H_2O$ = 464

| | C | H | N% |
|---|---|---|---|
| Calculé | 69,89 | 6,52 | 9,06 |
| Trouvé | 69,44 | 6,93 | 8,87 |

En CCM, Rf = 0,32 (solvant : $CH_2Cl_2$ + 15% éthanol) ; révélation par U.V. : fluorescence jaune.

Exemple VI

($\gamma$-diméthylaminopropyl)-8-diphényl-1,3 dihydro-1,8 (dibenzo [b,f] pyrazolo [3,4-d])azépine (14, Id, R = $(CH_2)_3-N(CH_3)_2$ ; $R_4=R_5=H$ ; $R_7=R_8=C_6H_5$).

On dissout 0,5 g de la dibenzopyrazolo 5H azépine 7 dans 250 ml de nitrobenzène et on chauffe la solution au bain d'huile sous agitation pendant 36 heures à 150-155°C. On distille ensuite le nitrobenzène sous pression réduite. Le produit déshydrogéné cristallise. On le redissout dans le minimum d'isopropanol, on ajoute de l'acide chlorhydrique jusqu'à réaction acide ; le chlorhydrate cristallise. On le sépare par essorage, puis on le recristallise de l'isopropanol. Le produit pur fond à 245° C, Rdt = 60 %.

Analyse $C_{32}H_{31}N_4Cl$, 0,5 $H_2O$ = 516

| | C | H | N% |
|---|---|---|---|
| Calculé | 74,48 | 6,25 | 10,86 |
| Trouvé | 74,40 | 6,55 | 10,64 |

En CCM, Rf = 0,3 ($CH_2Cl_2$ + 15 % éthanol) ; révélation par U.V. : fluorescence bleue.

Selon le même procédé, on peut obtenir les dibenzo [b,f] pyrazolo [3,4-d] dihydroazépines (Id) et les dibenzo [b,f] isoxazolo [4,5-d] 8H azépines (Ic).

Préparation des matières premières déjà connues dans la littérature

1-( γ -diméthylaminopropyl)-5 dibenzo [b,f] 5H azépine, 21

19,3 g d'iminostilbène sont dissous dans 600 ml de toluène sec. On ajoute 3,9 g d'amidure de sodium et on maintient la suspension sous agitation tout en chauffant 10 h au reflux du solvant. On ajoute alors une solution de 0,1 mole de chlorure de N,N-diméthyl-aminopropyle (libéré du chlorhydrate par l'ammoniaque et extrait au benzène) dans le benzène et on porte le mélange au reflux pendant 20 heures. Après refroidissement, on ajoute 250 ml d'eau et on épuise la phase benzénique à trois reprises avec 100 ml d'acide chlorhydrique à 18%. Les extraits acides sont neutralisés au carbonate de potassium solide et la base est extraite au chlorure de méthylène. Le solvant est séché sur sulfate de sodium, puis évaporé sous pression réduite. La base cristallise. Elle est dissoute dans le minimum d'isopropanol. On ajoute une solution saturée de gaz chlorhydrique dans l'éther anhydre. Le chlorhydrate précipite. On recristallise dans l'isopropanol : 20 g ; Rdt = 71% ; F = 175° C.

Analyse $C_{19}H_{23}N_2Cl = 315$

| | C | H | N% |
|---|---|---|---|
| Calculé | 72,44 | 7,36 | 8,89 |
| Trouvé | 72,60 | 7,42 | 9,07 |

En CCM, Rf = 0,37 ($CHCl_3$ + 15 % méthanol) ; révélation par U.V. : fluorescence jaune.

De la même façon on obtient :

- la (méthyl-2 diméthylamino-3 propyl)-5 dibenzo [b,f] 5H azépine ; 22
F = 204°C sous forme de chlorhydrate ; Rdt = 55 %.

Analyse $C_{20}H_{25}N_2Cl = 329$

| | C | H | N% |
|---|---|---|---|
| Calculé | 73,01 | 7,66 | 8,52 |
| Trouvé | 72,99 | 7,69 | 8,47 |

En CCM, Rf = 0,40 ($CHCl_3$ + 15 % méthanol) ; révélation par U.V. : fluorescence jaune ;

- la (β-diméthylaminoéthyl)-5 dibenzo [b,f] 5H azépine ; 29 sous forme de chlorhydrate F = 186° C ; Rdt = 86 %

Analyse $C_{18}H_{21}N_2Cl$ = 301

| | C | H | N% |
|---|---|---|---|
| Calculé | 71,82 | 7,03 | 9,31 |
| Trouvé | 71,69 | 7,04 | 9,32 |

En CCM, Rf = 0,45 ($CHCl_3$ + 15 % méthanol) ; révélation par U.V. : fluorescence jaune.

- la ( 1'-[β -hydroxyéthyl-4')pipérazinyl] -3 propyl)-5 dibenzo [b,f] 5H azépine, par condensation de l'iminostilbène sodé avec le bromo-1 chloro-3 propane, suivie de celle de la N-β-acétoxyéthylpipérazine, puis saponification du chlorhydrate obtenu ; F = 100°C ; dichlorhydrate, F = 230°C

Analyse (base) $C_{23}H_{29}N_3O$ = 363,5

| | C | H | N% |
|---|---|---|---|
| Calculé | 75,99 | 7,98 | 11,55 |
| Trouvé | 76,11 | 7,89 | 11,69 |

ETUDE DES PROPRIETES PHARMACOLOGIQUES ET TOXICOLOGIQUES DES COMPOSES SELON L'INVENTION

A. ETUDE TOXICOLOGIQUE

TOXICITE AIGUE CHEZ LA SOURIS

a) Matériel et méthode

Les produits ont été administrés par voie intrapéritonéale à des souris femelles dont le poids variait de 18 à 30 g.

Les animaux injectés ont été maintenus dans une salle thermostatée, nourris et abreuvés à volonté.

La mortalité a été relevée à 1 heure, 4 heures, 24 heures et 48 heures.

b) Résultats

Les résultats des essais de toxicité aiguë chez la Souris, qui sont rapportés dans les tableaux qui suivent, témoignent donc de la très grande tolérance des animaux éprouvés vis-à-vis d'un nombre très représentatif des substances selon l'invention.

Les résultats obtenus dans les essais de toxicité à moyen terme chez le Rat et de toxicité cardiovasculaire chez le Chien font également apparaître une très grande innocuité des substances selon l'invention, comme en témoignent plus particulièrement les résultats obtenus avec l'un des représentants des classes de produits revendiqués, celui de l'exemple I.

TOXICITE AIGUE

| Composés N° | Dose mg/kg | Nombre Souris | Mortalité en 1 heure | Mortalité en 48 h. | Mortalité % | DL 50 mg/kg |
|---|---|---|---|---|---|---|
| 3 | 100<br>150<br>200<br>250 | 5<br>5<br>5<br>5 | 0<br>0<br>2<br>5 | 0<br>0<br>2<br>5 | 0<br>0<br>40<br>100 | 200 |
| 8 | 5<br>50<br>100<br>150<br>250 | 5<br>5<br>5<br>3<br>5 | 0<br>0<br>0<br>1<br>0 | 0<br>0<br>0<br>1<br>0 | 0<br>0<br>0<br>20<br>0 | >250 |
| 2 | 10<br>50<br>75<br>100<br>200 | 5<br>10<br>5<br>5<br>5 | 0<br>0<br>0<br>3<br>4 | 0<br>0<br>0<br>3<br>5 | 0<br>0<br>0<br>60<br>100 | 95 |
| 7 | 250<br>500<br>900 | 10<br>5<br>2 | 0<br>0<br>1 | 0<br>0<br>1 | 0<br>0 | >500 |
| 4 | 100<br>300 | 5<br>4 | 0<br>4 | 0<br>4 | 0<br>100 | 175 |
| 9 | 150<br>300 | 5<br>5 | 0<br>0 | 0<br>0 | 0<br>0 | >300 |
| 6 | 500<br>750 | 5<br>5 | 0<br>0 | 0<br>0 | 0<br>0 | > 750 |
| 12 | 125<br>250 | 5<br>5 | 0<br>0 | 0<br>0 | 0<br>0 | > 250 |
| 1 | 50<br>100 | 5<br>5 | 0<br>0 | 0<br>0 | 0<br>0 | > 100 |

TOXICITE AIGUE

| Composés N° | Dose mg/kg | Nombre Souris | Mortalité en 1 heure | Mortalité en 48 heures | Mortalité % | DL/50 mg/kg |
|---|---|---|---|---|---|---|
| 21 | 50<br>75<br>125<br>250 | 4<br>5<br>5<br>5 | 0<br>0<br>5<br>5 | 0<br>0<br>5<br>5 | 0<br>0<br>100<br>100 | 100 |
| 20 | 50<br>100<br>200 | 9<br>5<br>5 | 0<br>3<br>5 | 0<br>3<br>5 | 0<br>60<br>100 | 75 |
| 22 | 25<br>50<br>100 | 5<br>5<br>5 | 0<br>0<br>4 | 0<br>1<br>5 | 0<br>20<br>100 | 75 |
| 11 | 250<br>450<br>1000 | 5<br>5<br>5 | 0<br>0<br>0 | 0<br>0<br>0 | 0<br>0<br>0 | >1000 |
| 5 | 75<br>125<br>200<br>250 | 5<br>5<br>5<br>5 | 1<br>2<br>5<br>5 | 1<br>2<br>5<br>5 | 20<br>40<br>100<br>100 | 125 |
| 10 | 200<br>300<br>750<br>1500 | 5<br>5<br>5<br>1 | 0<br>0<br>0<br>0 | 0<br>0<br>0<br>1 | 0<br>0<br>0 | >750 |

TOXICITE AIGUE

| Composés N° | Dose mg/kg | Nombre Souris | Mortalité en 1 heure | Mortalité en 48 heures | Mortalité % | DL/50 mg/kg |
|---|---|---|---|---|---|---|
| 16 | 50<br>100<br>250<br>500<br>1000 | 5<br>5<br>5<br>5<br>5 | 0<br>0<br>0<br>0<br>0 | 0<br>0<br>1<br>0<br>0 | 0<br>0<br>20<br>0<br>0 | >1000 |
| 18 | 50<br>100<br>150<br>200<br>250 | 5<br>5<br>5<br>5<br>5 | 0<br>0<br>0<br>5<br>4 | 0<br>0<br>0<br>5<br>5 | 0<br>0<br>0<br>100<br>100 | 225 |
| 17 | 50<br>100<br>150<br>200<br>250 | 5<br>5<br>5<br>5<br>5 | 0<br>0<br>0<br>5<br>4 | 0<br>0<br>0<br>5<br>5 | 0<br>0<br>0<br>100<br>100 | 175 |
| 19 | 50<br>100<br>250<br>500 | 5<br>10<br>5<br>5 | 0<br>0<br>1<br>5 | 0<br>0<br>3<br>5 | 0<br>0<br>60<br>100 | 175 |
| 14 | 50<br>100<br>250<br>500 | 5<br>5<br>5<br>5 | 0<br>0<br>2<br>3 | 0<br>0<br>4<br>5 | 0<br>0<br>80<br>100 | 200 |
| 13 | 50<br>100<br>250<br>400<br>750 | 5<br>10<br>5<br>5<br>5 | 0<br>0<br>0<br>3<br>2 | 0<br>0<br>0<br>3<br>5 | 0<br>0<br>0<br>60<br>100 | 350 |
| 15 | 50<br>100<br>250<br>300 | 5<br>5<br>5<br>5 | 0<br>0<br>2<br>0 | 0<br>0<br>4<br>4 | 0<br>0<br>80<br>80 | 175 |

TOXICITE A MOYEN TERME CHEZ LE RAT

Cette étude a eu pour but de rechercher les effets éventuels du composé 2 sur les différents paramètres biologiques hématologi-ques et histologiques du rat après traitement per os à raison de 2,10 et 50 mg/kg pendant 5 semaines.

Les résultats obtenus n'ont révélé aucune modification de ces paramètres.

Aucune mortalité n'a été relevée au cours de ces essais.

TOXICITE CARDIOVASCULAIRE CHEZ LE CHIEN

L'étude du composé 2 a été effectuée sur 7 chiens anesthésiés à des doses allant de 4 à 20 mg/kg en perfusion lente.

Aux doses les plus faibles 4,5, 10 mg/kg, les paramètres cardiovasculaires étudiés (pression artérielle périphérique, pression ventriculaire gauche, électrocardiogramme, etc..) ne sont pas modifiés 30 minutes après l'injection.

Les essais menés comparativement à l'imipramine (DCI) confirment une plus faible toxicité du composé 2. Plus particulièrement, il faut noter le retour vers les valeurs de départ des paramètres enregistrés, dès l'arrêt de l'injection, ce qui laisse préjuger d'une faible fixation cardiaque alors que cette propriété n'a pas été constatée avec l'imipramine.

Ces composés seraient donc doués d'une toxicité sur le système cardiovasculaire beaucoup plus faible que l'imipramine considérée comme l'antidépresseur de référence.

B. ETUDE DE L'ACTIVITE PHARMACOLOGIQUE

I. Etude de l'activité antidépressive

1. Hypothermie réserpinique.

a) Méthode

Les souris reçoivent 17 heures avant l'administration des produits à étudier, la réserpine par voie intrapéritonéale, à raison de 5 mg/kg, qui donne des résultats reproductibles et homogènes.

On note ensuite les températures témoins (réserpine), puis les températures 1, 2, 3 et 4 heures après l'administration des

produits étudiés.

L'hypothermie à la réserpine par cette technique est importante, de l'ordre de 15° C.

Les animaux sont répartis en lots de 4 animaux et reçoivent par voie intrapéritonéale la réserpine à une dose de 5 mg/kg. 17 heures après cette injection, on mesure la température corporelle de l'animal à l'aide d'une sonde thermocouple introduite dans le rectum; chaque lot reçoit ensuite la substance par voie intrapéritonéale. Le lot témoin reçoit la même quantité de solvant et par la même voie. La température corporelle est ensuite mesurée toutes les heures pendant 4 heures.

b) Expression des résultats

Les résultats sont rassemblés dans un tableau récapitulatif où sont indiqués :

- la température moyenne pour chaque lot relevée 17 heures après l'injection de la réserpine,
- la moyenne des variations de température pour chaque lot relevées 1,2, 3 et 4 heures après l'injection de la substance étudiée,
- la somme des variations de températures pendant 4 heures,
- le pourcentage d'augmentation de la température pour chaque produit par rapport aux témoins.

c) Interprétation des résultats

La réserpine administrée par voie intrapéritonéale à raison de 5 mg/kg produit chez la Souris une hypothermie importante d'environ I5°C (37°C à 22°C).

Les produits retenus entraînent une augmentation de température après 4 heures, supérieure ou égale à 70% de la température moyenne des témoins (le lot des témoins comprenait 23 souris).

Les produits les plus actifs présentent une activité antiréserpine importante à la dose de 50 mg/kg.

Toutes les substances présentent une activité supérieure à celle de l'amineptine, prise comme substance de comparaison.

ACTIVITE ANTIRESERPINE : HYPOTHERMIE

| Composés N° | Dose mg/kg | Tempé-rature T = 0 | T =1h | T= 2h | T=3h | T=4h | Augmentation de tempéra-ture en 4 h | % d'augmenta-tion par rap-port aux témoins |
|---|---|---|---|---|---|---|---|---|
| 3 | 50 | 21°3 | + 2,6 | +0,9 | +0,9 | +0,1 | + 4,5 | 125 |
| 2 | 5 | 22°2 | + 1,4 | +1,6 | +0,7 | +1,4 | + 5,1 | 155 |
| 7 | 100 | 22°8 | + 2,8 | +1,1 | +0,7 | +0,1 | + 4,5 | 125 |
| 9 | 50 | 22°3 | + 1,7 | +0,8 | +0,6 | +0,1 | + 3,2 | 60 |
| 6 | 150 | 22°3 | + 1,9 | +0,7 | +0,6 | +0,4 | + 3,6 | 80 |
| 12 | 50 | 22°3 | + 1,1 | +0,4 | +0,3 | +0,4 | + 2,2 | 10 |
| 1 | 15 | 22°4 | + 1,3 | +0,5 | +0,4 | +0,7 | + 2,9 | 45 |
| Témoin | | 23° | + 1,2 | +0,5 | +0,2 | +0,1 | + 2 | |

ACTIVITE ANTIRESERPINE : HYPOTHERMIE

| Composés N° | Dose mg/kg | Température T = 0 | T = 1h | T = 2h | T= 3h | T = 4h | Augmentation de température en 4h | % d'augmentation par rapport aux témoins |
|---|---|---|---|---|---|---|---|---|
| 21 | 50 | 22°4 | + 2 | -0,1 | +0,2 | +0,5 | + 2,6 | 30 |
| 20 | 50 | 22°6 | + 5,1 | +2,7 | +2 | +1 | +10,8 | 440 |
| 22 | 25 | 22°6 | + 1,8 | +0,5 | +1 | +0,4 | + 3,7 | 85 |
| 11 | 150 | 23° | + 2,1 | +0,4 | +0,5 | +0,7 | + 3,7 | 85 |
| 10 | 150 | 22°4 | + 1,7 | +2,4 | +1,4 | +0,6 | + 6,1 | 205 |
| Témoin | | 23° | + 1,2 | +0,5 | +0,2 | +0,1 | + 2 | |

ACTIVITE ANTIRESERPINE : HYPOTHERMIE

| Composés N° | Dose mg/kg | Température T = 0 | T=1h | T=2h | T=3h | T=4h | Augmentation de température en 4 h | % d'augmentation par rapport aux témoins |
|---|---|---|---|---|---|---|---|---|
| 18 | 50 | 22°5 | +2,8 | 0 | -0,3 | 0 | + 2,5 | 25 |
| | 150 | 22°3 | +1,6 | +1,9 | +2,1 | -0,1 | + 5,5 | 175 |
| 17 | 50 | 22°8 | +1,4 | +0,8 | -0,6 | -0,1 | + 1,5 | 0 |
| | 150 | 22°6 | +2,5 | +5,2 | +0,7 | -0,4 | + 8 | 300 |
| 19 | 150 | 22°8 | +3,4 | +0,7 | +0,7 | -0,6 | + 4,2 | 110 |
| 14 | 150 | 22°6 | +1,5 | +0,6 | +0,6 | +0,1 | + 2,8 | 40 |
| 13 | 200 | 21°5 | +1,9 | +1 | +0,5 | 0 | + 3,4 | 70 |
| Témoin | | 23° | +1,2 | +0,5 | +0,2 | +0,1 | + 2 | 0 |
| Amineptine | 80 | 22° | +1,5 | +1,6 | +1 | +0,1 | + 4,2 | |

2. Méthode à la tétrabénazine

On a également étudié les propriétés protectrices des substances envers les effets (hypothermie et ptosis) de la tétrabénazine injectée par voie intraveineuse à la dose de 32 mg/kg. Ce test permet de révéler également une activité antidépressive avec l'avantage d'une lecture plus rapide qu'avec la réserpine.

Les produits sont injectés 30 minutes avant l'administration de tétrabénazine.

Les températures sont relevées aux temps : 0, 30 minutes, 60 minutes, 90 minutes et 120 minutes.

α. Hypothermie à la tétrabénazine

1. Mode opératoire

Les souris sont réparties par lots de 5.

On administre les produits à étudier en intrapéritonéale 30 minutes avant l'injection intraveineuse de tétrabénazine à raison de 32 mg/kg.

Les températures sont relevées avant l'injection de tétrabénazine, 30 minutes, 60 minutes, 90 minutes et 120 minutes après.

Le lot témoin comprend 20 souris.

2. Résultats

Les résultats apparaissent dans le tableau ci-joint.

HYPOTHERMIE A LA TETRABENAZINE

| Composés N° | Dose mg/kg | Température 30 minutes après administration du produit (°C) | Température après tétrabénazine | | | | | % de protection |
|---|---|---|---|---|---|---|---|---|
| | | | 30 minutes | 60 minutes | 90 minutes | 120 minutes | Total | |
| 3 | 50 | 34,4 | -0,9 | -1,2 | -2,6 | -0,4 | -5,1 | 40 |
| 2 | 5 | 37,6 | -3,2 | -2,6 | -1,3 | -1,3 | -8,3 | 3 |
| 7 | 100 | 35,3 | -3,2 | -1,3 | -1 | -0,8 | -6,3 | 26 |
| 20 | 50 | 35,6 | -3,1 | -0,2 | -0,3 | -1 | -4,6 | 46 |
| 22 | 25 | 32,5 | -3,5 | -0,1 | -0,6 | -0,1 | -4,3 | 50 |
| 10 | 150 | 34 | -1,7 | -1,9 | +0,6 | -1 | -4 | 53 |
| 18 | 50 | 35,2 | -3,1 | -1,8 | -1,9 | -1,1 | -7,9 | 8 |
| 13 | 200 | 35,6 | -3,4 | -1,9 | -1,1 | -0,9 | -7,3 | 15 |
| Témoin | | 37 | -3,4 | -2,6 | -1,9 | -0,7 | -8,6 | |
| Amineptine | 80 | 36,1 | -1,8 | -1,8 | -1,1 | -0,2 | -4,2 | 51 |

β. Ptosis à la tétrabénazine

1. Mode opératoire

Les souris sont réparties par lots de 5.

On administre les produits à étudier en intrapéritonéale 30 minutes avant l'injection intraveineuse de tétrabénazine à raison de 32 mg/kg.

Le ptosis est noté avant l'injection de tétrabénazine, 30 minutes, 60 minutes, 90 minutes et 120 minutes après.

On a adopté la notation suivante :

- 0 : pour un oeil ouvert,
- 1 : pour un oeil fermé au quart,
- 2 : pour un oeil fermé à moitié,
- 3 : pour un oeil fermé aux trois-quarts,
- 4 : pour un oeil fermé complètement.

Le lot témoin comprend 20 souris.

2. Résultats

Les résultats apparaissent dans le tableau ci-joint.

PTOSIS A LA TETRABENAZINE

| Composé N° | Dose mg/kg | Ptosis T = 0 | Ptosis 30 minutes | Ptosis 60 minutes | Ptosis 90 minutes | Ptosis 120 minutes | Total | % de protection |
|---|---|---|---|---|---|---|---|---|
| 3 | 50 | 0 | 1,6 | 5,2 | 6,8 | 6,8 | 20,4 | 32 |
| 2 | 5 | 0 | 4,8 | 7,8 | 8 | 8 | 28,6 | 5 |
| 7 | 100 | 0 | 3,8 | 7 | 7 | 8 | 25,8 | 14 |
| 20 | 50 | 0 | 2,2 | 3,4 | 3,2 | 3,6 | 12,2 | 60 |
| 22 | 25 | 0 | 5,6 | 6,8 | 6,8 | 7,6 | 26,8 | 11 |
| 10 | 150 | 0 | 1 | 6 | 5,8 | 8 | 20,8 | 31 |
| 18 | 50 | 0 | 3,6 | 5,8 | 6,4 | 7,2 | 23 | 24 |
| 19 | 100 | 0 | 5 | 6,3 | 6,7 | 7,3 | 25,3 | 16 |
| 13 | 200 | 0 | 4 | 6,8 | 7,6 | 7,5 | 25,9 | 14 |
| Témoin | | 0 | 6,6 | 7,6 | 8 | 8 | 30,2 | |

II. Etude de l'activité analgésique (technique à la phénylbenzoquinone).

Ce test consiste à rechercher une éventuelle protection vis-à-vis des crampes ou contorsions abdominales provoquées chez la Souris par injection intrapéritonéale de phénylbenzoquinone.

Les souris sont réparties au hasard par lots de 4 dans des cristallisoirs.

Le composé 2 a été injecté par voie intrapéritonéale, 30 minutes avant l'injection de la phénylbenzoquinone.

Les crampes abdominales sont comptées pour chaque souris pendant 30 minutes à partir de la quinzième minute qui suit l'injection de l'agent algogène.

Résultats

| Composé 2 mg/kg/IP | phényl-benzoquinone en mg/kg/IP | Nombre de crampes en 30 minutes | % de protection |
|---|---|---|---|
| 0 | 10 | 140 | 0 |
| 20 | 10 | 35 | 70 |
| 50 | 10 | 0 | 100 |

Le composé 2 possède une action protectrice importante envers le syndrome douloureux à la phénylbenzoquinone.

III. Etude de l'action sur le système nerveux autonome.

Etude de l'action spasmolytique.

a) Méthode

Les essais ont été effectués sur l'Iléon de cobaye maintenu en survie dans une solution de Tyrode oxygénée et thermostabilisée à 37° C suivant le méthode de MAGNUS.

L'action spasmolytique du composé 2 a été étudiée en

opposition avec les propriétés spasmogènes de l'acétylcholine, du chlorure de baryum, de l'histamine et de la sérotonine.

b) Résultats

1°) Effet anticholinergique

Ces essais ont été effectués sur six préparations. L'effet inhibiteur du composé 2 a été recherché à l'encontre des propriétés contracturantes de l'acétylcholine utilisée à la concentration de 3 à 6 x $10^{-5}$ mg/ml pour une cuve de 30 ml.

La DE 50 déterminée sur ces six préparations est de 5 x $10^{-4}$ mg/ml.

2°) Effet antichlorure de baryum

Ces essais ont été effectués sur douze préparations. Six ont été utilisées pour la recherche d'inhibition de la contraction barytique dite curative, les six autres pour les essais dits "préventif baryum".

La concentration de $BaCl_2$ utilisée comme agent contracturant est de 6 x $10^{-2}$ mg/ml.

Le composé 2 entraîne une inhibition de 50% de l'effet spasmogène pour une concentration de 5 x $10^{-4}$ mg/ml lors des essais préventifs et de 1 x $10^{-3}$ mg/ml lors des essais curatifs.

3°) Effet antihistaminique

Ces essais ont été pratiqués sur cinq préparations. L'histamine a été utilisée à la concentration de 0,5 à 3 x $10^{-5}$mg/ml.

La DE 50 du composé 2 envers le spasme provoqué par l'histamine se situe aux environs de 5 x $10^{-5}$ mg/ml.

4°) Effet antisérotonine

Quatre essais ont été pratiqués envers l'effet contracturant de la sérotonine utilisée à la concentration de 3 x $10^{-4}$mg/ml.

La DE 50 du composé 2 se situe entre 9 x $10^{-5}$mg/ml et 2 x $10^{-4}$ mg/ml.

REVENDICATIONS

1 - Les composés tétracycliques répondant à la formule générale I

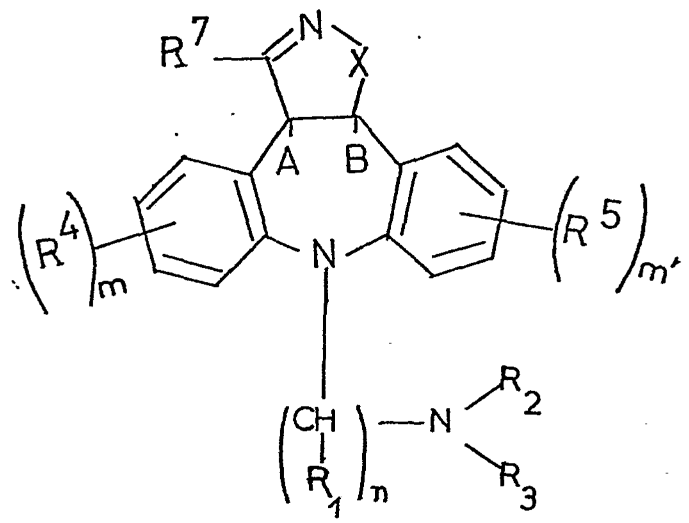

(I)

dans laquelle $R_1$ représente de l'hydrogène ou un radical alcoyle inférieur, n un nombre entier variant de 1 à 5,

$R_2$ représente de l'hydrogène, un radical alcoyle inférieur, un radical aralcoyle inférieur ou alcényle inférieur,

$R_3$ représente de l'hydrogène, un radical alcoyle inférieur, un radical aralcoyle inférieur ou alcényle inférieur, ou bien $R_2$ et $R_3$, ensemble, forment une chaîne alcoylène ayant de 2 à 6 atomes de carbone, éventuellement interrompue par 1 ou 2 hétéroatomes choisis dans le groupe constitué par un oxygène, un soufre et le radical N-$R_6$ ($R_6$ est de l'hydrogène, un radical alcoyle inférieur, un radical hydroxyalcoyle inférieur, un radical alcoxyalcoyle inférieur ou acyloxyalcoyle inférieur).

$R_4$ et $R_5$, distinctement l'un de l'autre, sont de l'hydrogène, un radical alcoyle inférieur, un halogène, un radical trifluorométhyle, un radical alcoxy inférieur, un groupement alcoylènedioxy, un hydroxy, un radical thio, un radical alcoyl thio inférieur, un radical trichlorométhoxy, un radical trifluorométhoxy, un radical trifluorométhyl thio, un radical amino, un radical alcoylamino inférieur, un radical arylamino, un radical alcoylamino sulfonyle inférieur, un radical morpholino sulfonyle, un radical aminosulfonyle, un cyano, un nitro, un carboxy, un radical alcoyloxycarbonyl, un groupe carbonamido, un sulfiny-

lé, un sulfonyle, un radical formyle ou un radical acyle inférieur.

$R_7$ est un radical alcoyle inférieur, un radical phényle ou un radical phényle éventuellement substitué par un radical $R_4$.

m et m', distinctement l'un de l'autre, varient de 1 à 3.

- A et B symbolisent 2 atomes d'hydrogène ou une double liaison carbone-carbone,

X représente de l'oxygène, ou le groupe N-$R_8$ ($R_8$ étant un radical phényle ou phényle substitué par un, deux ou trois substituants, ou un radical alcoyle inférieur) ;

2 - Composés selon la revendication 1, caractérisés par la formule I

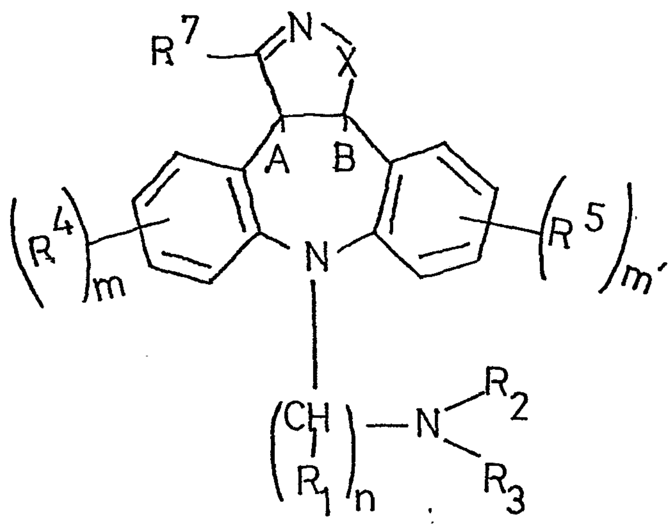

(I)

dans laquelle A et B ont les significations indiquées ci-dessus ; $R_4$ et $R_5$ sont de l'hydrogène ou représentent 1, 2 ou 3 substituants choisis parmi les alcoyle inférieur, halogénure, trifluorométhyle, alcoxy inférieur, amino et alcoyloxycarbonyle ; X est de l'oxygène, un groupe N-alcoyle inférieur ou N-phényle ou N-phényle substitué par 1, 2 ou 3 substituants, tels que ceux définis en rapport avec $R_4$ et $R_5$ ; $R_7$ est un radical alcoyle inférieur, un radical phényle ou un radical phényle substitué par 1, 2 ou 3 substituants choisis parmi ceux qui ont été définis à propos de $R_4$ et $R_5$, et $R_2$ et $R_3$ forment un groupe

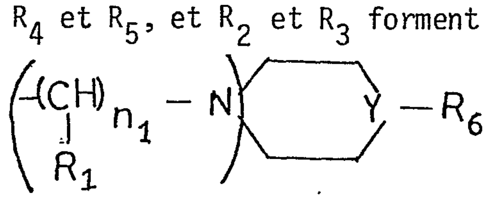

dans lequel Y est un atome d'azote ou d'oxygène et $R_6$ est de l'hydrogène, un radical alcoyle inférieur, un radical hydroxy - alcoyle inférieur, un radical alcoxy - alcoyle inférieur ou acyloxyalcoyle inférieur.

3 - Les composés selon la revendication 1 et caractérisés par la formule générale I dans laquelle A et B ont les significations indiquées précedemment ; X est de l'oxygène, un groupe N-alcoyle inférieur ou N-$C_6H_5$, $R_4$ et $R_5$ sont de l'hydrogène ; $R_7$ est de l'hydrogène ou un groupe phényle, le cas échéant substitué par un halogène.

4 - Les composés selon la revendication 1 et caractérisés par la formule générale I dans laquelle A, B ont les significations indiquées précédemment ; X est de l'oxygène, un groupe NH ou N-$C_6H_5$, $R_4$ et $R_5$ sont de l'hydrogène ; $R_7$ est de l'hydrogène ou un groupe phényle, le cas échéant substitué par un halogène, et dans le groupe de structure

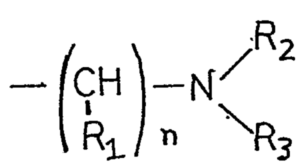

n varie de 2 à 5, avec au plus un seul des $R_1$ est un méthyle, les autres étant de l'hydrogène ; et $R_2$ et $R_3$ sont des groupes méthyle ou éthyle.

5 - Les composés selon la revendication 1 et caractérisés par la formule générale I dans laquelle A, B ont les significations précedemment indiquées ; X est de l'oxygène, un groupe N-alcoyle inférieur ou N-$C_6H_5$, $R_4$ et $R_5$ sont de l'hydrogène ; $R_7$ est de l'hydrogène ou un groupe phényle, le cas échéant substitué par un halogène, et le groupe aminoalcoylène a pour formule partielle

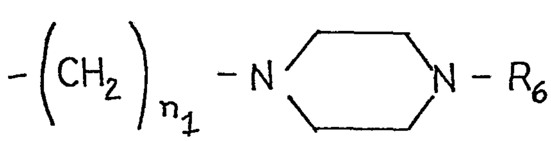

dans lequel $n_1$ vaut 1, 2 ou 3 et $R_6$ a la signification sus-indiquée, notamment hydroxyméthyle ou hydroxyéthyle.

6 - Les composés selon l'une quelconque des revendications 1 à 5, caractérisés par la formule générale Ia

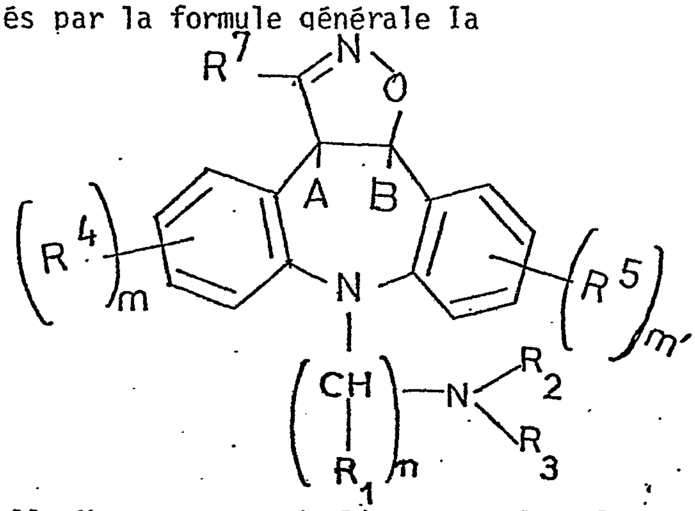

(Ia)

dans laquelle X représente de l'oxygène, A et B sont de l'hydrogène et les substituants $R_1$, $R_2$, $R_3$, $R_4$, $R_5$, $R_7$, d'une part, et les indices m et m', d'autre part, présentent les significations indiquées précédemment.

7 - Les composés selon l'une quelconque des revendications 1 à 5, caractérisés par la formule générale Ib

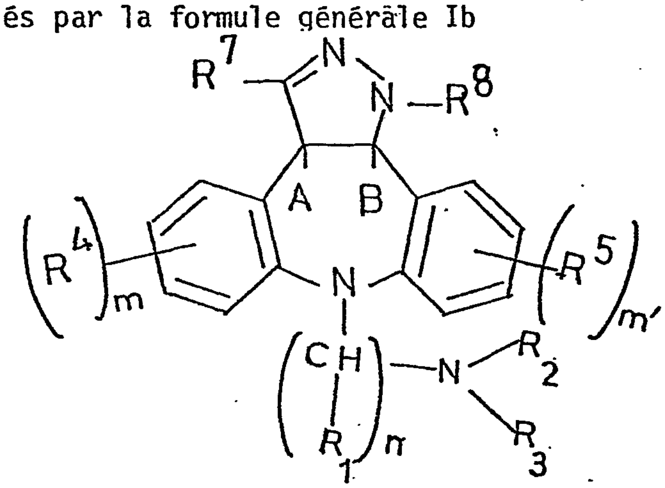

(Ib)

dans laquelle A et B sont chacun un atome d'hydrogène et les substituants $R_1$, $R_2$, $R_3$, $R_4$, $R_5$, R, $R_7$, $R_8$, d'une part, et les indices m et m', d'autre part, ont les significations sus-indiquées.

8 - Les composés selon l'une quelconque des revendications 1 à 5, caractérisés par la formule générale Ic

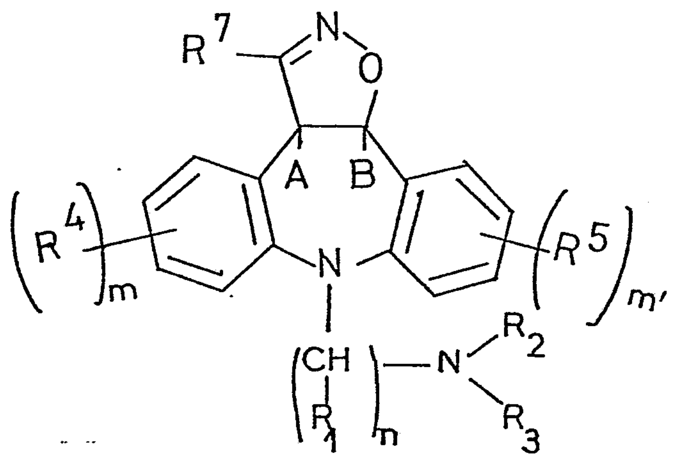

(Ic)

dans laquelle les substituants $R_1$, $R_2$, $R_3$, $R_4$, $R_5$, $R_7$ et les indices n, m et m' sont définis comme précédemment, A et B formant ensemble une double liaison carbone-carbone.

9 - Les composés selon la revendication 1, caractérisés par la formule générale $I_d$

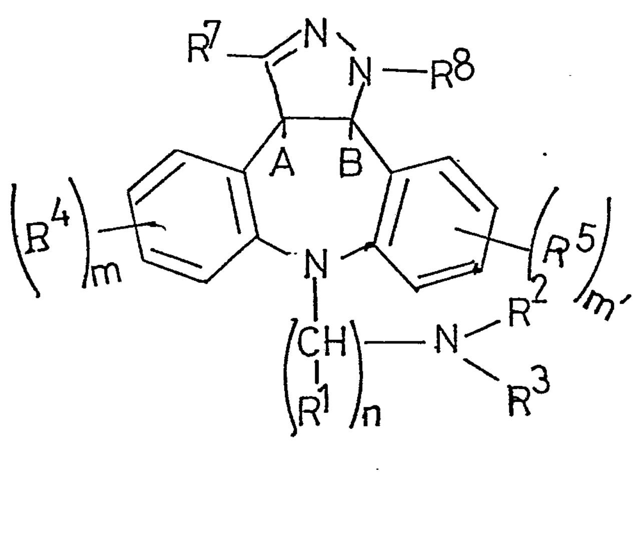

(Id)

dans laquelle les substituants $R_1$, $R_2$, $R_3$, $R_4$, $R_5$, $R_7$, $R_8$ et les indices n, m et m' sont définis comme précédemment et A et B représentent

ensemble une double liaison carbone-carbone.

10 - Les compositions pharmaceutiques pour le traitement des états dépressifs de toute nature, en tant qu'analgésiques et médicaments spasmolytiques, renfermant à titre de principe actif au moins un composé selon l'une quelconque des revendications 1 à 9 ou le composé de formule I dans laquelle, simultanément, A, B, $R_4$ et $R_5$ représentent de l'hydrogène, $R_7$ est un groupe phényle et X représente le groupe N-phényl, ledit composé se trouvant, le cas échéant, sous forme racémique ou dédoublée, ou un de ses sels d'addition avec un acide minéral ou organique, en association ou en mélange avec un excipient ou un véhicule inerte non toxique, pharmaceutiquement acceptable.

11 - Les compositions pharmaceutiques selon la revendication 10 dans lesquelles l'excipient ou le véhicule est un de ceux adaptés à l'administration par voie buccale, parentérale, rectale, sublinguale ou percutanée.

12 - Les compositions pharmaceutiques selon la revendication 10 dans lesquelles la teneur en principe actif s'échelonne de 25 à 150 mg par prise unitaire.

13 - Un procédé d'obtention des composés hydrogénés de formule générale $I_a$ et $I_b$

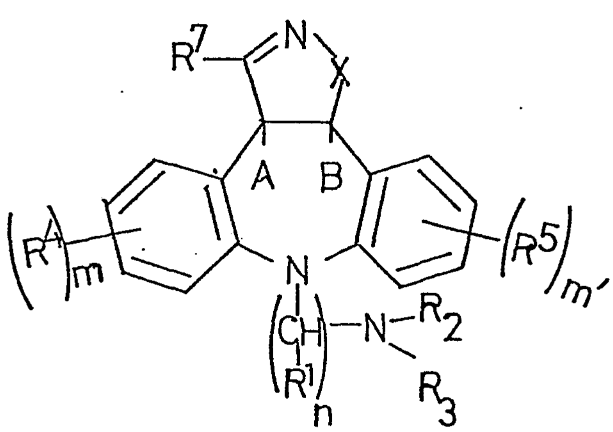

(Ia) + (Ib)

dans laquelle les substituants $R_1$, $R_2$, $R_3$, $R_4$, $R_5$, $R_7$, n, m et m' sont définis comme précédemment.

X représente de l'oxygène ou le groupe N-$R_8$ (dans lequel $R_8$ garde les significations fournies antérieurement), et A et B représentent chacun de l'hydrogène, caractérisé en ce que l'on fait réagir un iminostilbène de formule générale II

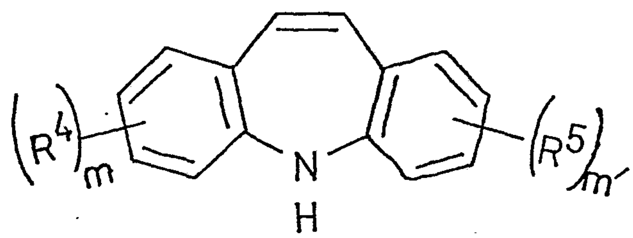

(II)

dans laquelle $R_4$, $R_5$, m et m' sont définis comme précédemment, avec un halogénure d'aminoalcoylène de formule générale III

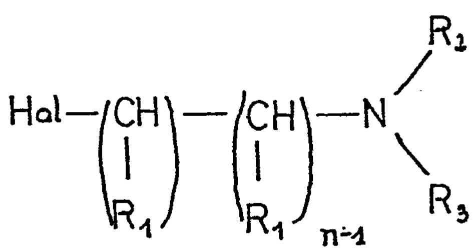

(III)

dans laquelle Hal représente du chlore ou du brome, en milieu basique, pour obtenir la N-(alcoylaminoalcoyl) dibenzazépine de formule générale IV

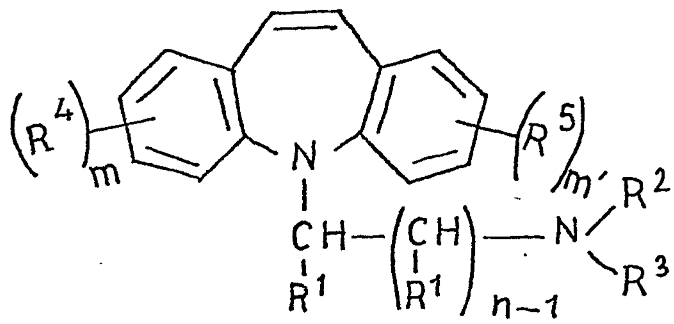

( IV )

dans laquelle la définition des substituants $R_1$, $R_2$, $R_3$, $R_4$, $R_5$, m, m' et n demeure celle fournie précédemment, puis on effectue sur celle-ci une réaction de condensation dipolaire 1-3, avec un oxyde de nitrile ou avec une nitrile-imine de formule

$$R_7 - \overset{\oplus}{C} = N - \overset{\ominus}{N} - R_8$$

ou

$$R_7 - \overset{\oplus}{C} = N \rightarrow \overset{\ominus}{O}$$

dans laquelle les substituants $R_7$ et $R_8$ sont définis comme précédemment, en milieu basique, à reflux d'un solvant inerte pour obtenir l'isoxazolo [4,5-d]-ou la pyrazolo [3,4-d]-dibenzazépine recherchée, de formule $I_a$ ou $I_b$ que l'on peut, si désiré,

- salifier par addition d'un acide minéral ou organique,
- dédoubler par salification à l'aide d'un acide organique, optiquement actif.
- ou déshydrogéner par chauffage avec un agent de déshydrogénation en un composé aromatique de formule générale

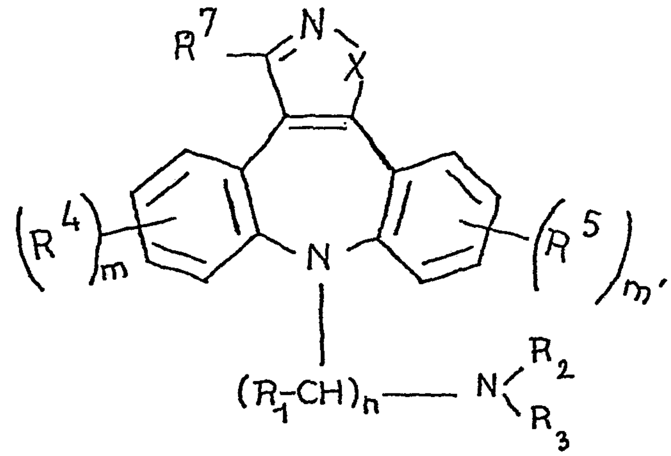

dans laquelle les substituants $R_1$, $R_2$, $R_3$, $R_4$, $R_5$, $R_7$, X, n, m et m' sont définis comme précédemment.

14 - Un procédé d'obtention des composés de formule générale $I_c$ et $I_d$ caractérisé en ce que l'on condense sur un imino-stilbène de formule générale II

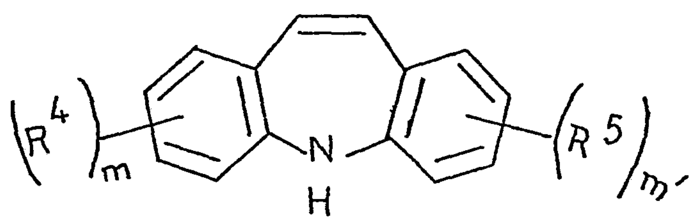

(II)

dans laquelle les substituants $R_4$, $R_5$, m et m' sont définis comme précédemment, selon une réaction d'addition dipolaire 1-3., un N-oxyde de nitrile de formule

$$R_7 - \overset{\oplus}{C} = N - \overset{\ominus}{O}$$

ou une nitrile-imine de formule $R_7 - \overset{\oplus}{C} = N - \overset{\ominus}{N} - R_8$ pour obtenir une isoxazolo[4,5-d]-ou une pyrazolo[3,4-d]-dibenzazépine de formule générale V

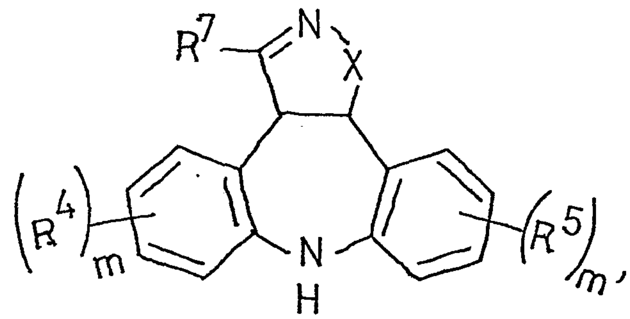

(V)

dans laquelle X représente de l'oxygène ou le groupe N-$R_8$ et dans laquelle les substituants $R_4$, $R_5$,$R_7$, m et m'sont définis comme précédemment, puis on soumet ce composé à une déshydrogénation au moyen d'un agent de déshydrogénation pour obtenir l'isoxazole ou le pyrazole correspondant de formule générale VI

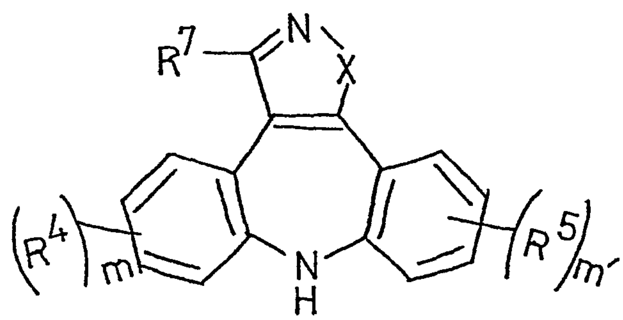

(VI)

dans laquelle les substituants $R_4$, $R_5$,$R_7$, X, m et m' sont définis comme précédemment, que l'on fait réagir avec un agent de métallation et on condense le dérivé métallique formé avec un halogénure d'aminoalcoylène de formule générale III

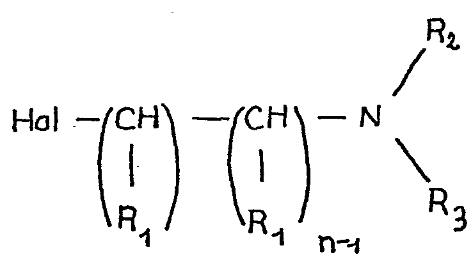

( III )

dans laquelle Hal est du brome ou du chlore et $R_1$, $R_2$, $R_3$, et n sont définis comme précédemment pour former le composé de formule générale $I_c$ ou $I_d$ cherché, que l'on peut, si désiré, salifier par addition d'un acide minéral ou organique ou dédoubler en ses isomères optiques lorsque la molécule comporte un atome de carbone chiral.

Office européen des brevets

**0063525**

Numéro de la demande

EP 82 40 0680

# RAPPORT DE RECHERCHE EUROPEENNE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | FR - A - 2 352 800 (AKZO)<br>* revendications 1 et 9 *<br>--------- | 1,10 | C 07 D 498/04<br>487/04<br>491/14<br>491/22<br>A 61 K 31/55//<br>(C 07 D 498/04<br>261/00<br>223/00)<br>(C 07 D 487/04<br>231/00<br>223/00) |
| | | | **DOMAINES TECHNIQUES RECHERCHES (Int. Cl. 3)** |
| | | | C 07 D 498/00<br>487/00<br>A 61 K 31/00 |

Le présent rapport de recherche a été établi pour toutes les revendications

| Lieu de la recherche | Date d'achèvement de la recherche | Examinateur |
|---|---|---|
| La Haye | 15-06-1982 | ALFARO |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82